# EUROPEAN PATENT APPLICATION

(11) **EP 1 849 481 A1**
(43) Date of publication of application: **31.10.2007**
(21) Application number: 06702035.4
(22) Date of filing: 06.01.2006
(51) Int. Cl.: A61K 47/10

(54) **COMPOSITION FOR EXTERNAL USE**

(30) Priority: 07.01.2005 JP 2005003210; 14.03.2005 JP 2005071144; 14.03.2005 JP 2005071145; 14.03.2005 JP 2005071231; 14.07.2005 JP 2005206231; 14.07.2005 JP 2005206232; 07.01.2005 JP 2005003209
(71) Applicant: ROHTO PHARMACEUTICAL CO., LTD., Osaka-shi, Osaka 544-8666 (JP)
(72) Inventor: HARADA, Ayako, shi 1-chome, Ikuno-ku, Osaka-shi, Osaka, (JP); HONMA, Yoichi, shi 1-chome, Ikuno-ku, Osaka-shi, Osaka, (JP); ABE, Masamichi, shi 1-chome, Ikuno-ku, Osaka-shi, Osaka, (JP)
(74) Representative: HOFFMANN EITLE
(86) International application number: PCT/JP2006/300105
(87) International publication number: WO 2006/073190

(57) **Abstract**

An object of the present invention is to provide a composition for external use in which the percutaneous absorbability of vitamin A, vitamin A derivative(s), vitamin C, specific vitamin C derivative(s), xanthine derivative(s), ubiquinone(s) and/or hyaluronic acid is improved.

The composition for external use is prepared by blending (i) a phospholipid and (ii) a mono- or oligo-glycol ether, together with (iv) at least one bioactive component selected from the group consisting of hyaluronic acid, hyaluronic acid derivatives, vitamin A, vitamin A derivatives, vitamin C, specific vitamin C derivatives, xanthine derivatives, ubiquinones, and salts thereof.

## Description

### TECHNICAL FIELD

The present invention relates to a composition for external use in which the percutaneous absorbability of bioactive component(s) is improved.

### BACKGROUND ART

Compositions for external use on the skin or mucosa are available in various forms such as patches, ointments, creams, lotions, solid preparations, etc. Compositions for external use are applied to affected parts of the skin by methods suitable for the forms of the compositions, but the percutaneous absorption of bioactive components contained in such compositions is reduced by the presence of the stratum corneum, which prevents external foreign substances from entering. Therefore, in compositions for external use, it is important that the bioactive components can efficiently permeate through the skin.

Various studies have been made to promote percutaneous absorption, and there have been reports on external preparations with improved percutaneous absorption, such as a liposome preparation containing a lecithin and a hydrophilic nonionic surfactant (Patent Document 1); a composition for external use containing a phospholipid and a specific polyhydric alcohol (Patent Document 2); etc.

Phospholipids naturally occur in animals and plants, such as soybeans, egg yolks, etc., and most phospholipids are highly safe components that can be used in food, and are also contained in medicines and cosmetics. Such phospholipids are known to have surface-active effects and moisturizing properties, and are also known to promote the percutaneous absorption of bioactive components when phospholipids are contained in compositions for external use. However, the percutaneous absorbability of bioactive components achieved by the effect of phospholipids are not yet satisfactory, and further improvement in percutaneous absorption-promoting effects is desired. Moreover, it has not been known what effects are achieved on the percutaneous absorption of bioactive components, by combining phospholipids and glycol ethers.

Among bioactive components used in compositions for external use, vitamin A, vitamin C, xanthine derivatives, ubiquinones, and hyaluronic acid have been reported to have various useful physiological activities. However, no formulations of compositions for external use are known which are suitable for improving the percutaneous absorption of such bioactive components and allowing such components to exhibit the desired useful effects.

In particular, it is known that hyaluronic acid, even when applied percutaneously, remains in the epidermis and does not readily permeate into the skin, because of its extremely large molecular weight of several thousand to five million. Known hyaluronic acid-containing compositions for external use therefore have a drawback in that the useful physiological effects of hyaluronic acid are not sufficiently obtained.
Patent Document 1: Japanese Unexamined Patent Application Publication No. 1987-95134
Patent Document 2: Japanese Unexamined Patent Application Publication No. 1998-194994

### DISCLOSURE OF THE INVENTION

### PROBLEMS TO BE SOLVED BY THE INVENTION

An object of the present invention is to provide a composition for external use in which bioactive components have excellent percutaneous absorbability. Another object of the present invention is to provide a composition for external use in which vitamin A, vitamin A derivative(s), vitamin C, specific vitamin C derivative(s), xanthine derivative(s), ubiquinone(s), hyaluronic acid, or hyaluronic acid derivative(s) have excellent percutaneous absorbability.

### MEANS FOR SOLVING THE PROBLEMS

The present inventors found that use of a composition for external use comprising (i) a phospholipid and (ii) a mono-or oligo-glycol ether can achieve the above object. Specifically, the inventors found that a composition for external use comprising specific proportions of phospholipid (i) and mono- or oligo-glycol ether (ii) can improve the percutaneous absorption of (iii) a bioactive component. Further, the inventors found that when a composition for external use containing (iv) vitamin A, vitamin A derivative(s), vitamin C, specific vitamin C derivative(s), xanthine derivative(s), ubiquinone(s), and/or hyaluronic acid as bioactive component(s) comprises the phospholipid (i) and mono- or oligo-glycol ether (ii), the percutaneous absorption efficiency of the above specific bioactive component(s) is remarkably enhanced. The present invention is accomplished by further improvements based on the above findings.

The present invention provides the following compositions for external use.

Item 1-1. A composition for external use comprising (i) a phospholipid and (ii) a mono- or oligo-glycol ether, wherein the proportion of mono- or oligo-glycol ether (ii) is at least 22 wt.% of the total amount of the composition.

Item 1-2. A composition according to item 1-1, further comprising (iii) a bioactive component.

Item 1-3. A composition according to item 1-1, wherein the phospholipid (i) is at least one member selected from the group consisting of glycerophospholipids and sphingophospholipids.

Item 1-4. A composition according to item 1-1, wherein the phospholipid (i) is a hydrogenated phospholipid.

Item 1-5. A composition according to item 1-1, wherein the phospholipid (i) is a non-hydrogenated phospholipid.

Item 1-6. A composition according to item 1-1, wherein the phospholipid (i) is a lecithin.

Item 1-7. A composition according to item 1-5, wherein the lecithin has an iodine value of 10 to 50.

Item 1-8. A composition according to item 1-1, wherein the mono- or oligo-glycol ether (ii) is a compound represented by Formula (1):

R₁-O- (A-O)ₙ-R₂ (1)

wherein R₁ and R₂ are the same or different, and each independently represent a C₁₋₆ alkyl group, a C₂₋₆ alkenyl group, an aryl group, an acetyl group, or a hydrogen atom, provided that at least one of R₁ and R₂ represents a C₁₋₆ alkyl group, a C₂₋₆ alkenyl group, or an aryl group;
A represents a C₂₋₄ alkylene group; and
n represents an integer of 1 to 4.

Item 1-9. A composition according to item 1-1, wherein the mono- or oligo-glycol ether (ii) is at least one member selected from the group consisting of ethylene glycol monoethers, ethylene glycol diethers, diethylene glycol monoethers, diethylene glycol diethers, triethylene glycol monoethers, triethylene glycol diethers, tetraethylene glycol monoethers, tetraethylene glycol diethers, propylene glycol monoethers, propylene glycol diethers, dipropylene glycol monoethers, dipropylene glycol diethers, tripropylene glycol monoethers, tripropylene glycol diethers, butylene glycol monoethers, and butylene glycol diethers.

Item 1-10. A composition according to item 1-1, wherein the proportion of phospholipid (i) is 0.01 to 15 wt.% of the total amount of the composition.

Item 1-11. A composition according to item 1-1, wherein the phospholipid (i) is a hydrogenated lecithin and/or a non-hydrogenated lecithin.

Item 1-12. A composition according to item 1-2, wherein the bioactive component (iii) is at least one member selected from the group consisting of vitamin compounds, skin-whitening agents, anti-wrinkle agents, anti-inflammatory analgesics, antifungals, steroids, hair restorers, slimming agents, local anesthetics, antipruritics, antimicrobials, antivirals, keratin softeners, moisturizers, astringents, antioxidants, and hair growth inhibitors.

Item 1-13. A composition according to item 1-1, which is a liquid crystal composition for external use.

Item 1-14. A composition according to item 1-1, which is a microemulsion composition for external use.

Item 1-15. A composition according to item 1-1, which is a liposome composition for external use.

Item 1-16. A composition according to item 1-1, which is a pharmaceutical composition.

Item 1-17. A composition according to item 1-1, which is a cosmetic composition.

The present invention also provides the following compositions for external use.

Item 2-1. A composition for external use comprising (i) a phospholipid in a proportion of 0.01 to 8 wt.%, and (ii) a mono- or oligo-glycol ether in a proportion of 0.01 to 6.5 wt.%, based on the total amount of the composition.

Item 2-2. A composition according to item 2-1, further comprising (iii) a bioactive component.

Item 2-3. A composition according to item 2-1, wherein the phospholipid (i) is at least one member selected from the group consisting of glycerophospholipids and sphingophospholipids.

Item 2-4. A composition according to item 2-1, wherein the phospholipid (i) is a hydrogenated phospholipid.

Item 2-5. A composition according to item 2-1, wherein the phospholipid (i) is a non-hydrogenated phospholipid.

Item 2-6. A composition according to item 2-1, wherein the phospholipid (i) is a lecithin.

Item 2-7. A composition according to item 2-5, wherein the lecithin has an iodine value of 10 to 50.

Item 2-8. A composition according to item 2-1, wherein the mono- or oligo-glycol ether (ii) is a compound represented by Formula (1):

R₁-O- (A-O)ₙ-R₂ (1)

wherein R₁ and R₂ are the same or different, and each independently represent a C₁₋₆ alkyl group, a C₂₋₆ alkenyl group, an aryl group, an acetyl group, or a hydrogen atom, provided that at least one of R₁ and R₂ represents a C₁₋₆ alkyl group, a C₂₋₆ alkenyl group, or an aryl group;
A represents a C₂₋₄ alkylene group; and
n represents an integer of 1 to 4.

Item 2-9. A composition according to item 2-1, wherein the mono- or oligo-glycol ether (ii) is at least one member selected from the group consisting of ethylene glycol monoethers, ethylene glycol diethers, diethylene glycol monoethers, diethylene glycol diethers, triethylene glycol monoethers, triethylene glycol diethers, tetraethylene glycol monoethers, tetraethylene glycol diethers, propylene glycol monoethers, propylene glycol diethers, dipropylene glycol monoethers, dipropylene glycol diethers, tripropylene glycol monoethers, tripropylene glycol diethers, butylene glycol monoethers, and butylene glycol diethers.

Item 2-10. A composition according to item 2-1, wherein the ratio of mono- or oligo-glycol ether (ii) is 0.01 to 70 parts by weight per part by weight of phospholipid (i).

Item 2-11. A composition according to item 2-1, wherein the phospholipid (i) is a hydrogenated lecithin and/or a non-hydrogenated lecithin.

Item 2-12. A composition according to item 2-2, wherein the bioactive component (iii) is at least one member selected from the group consisting of vitamin compounds, skin-whitening agents, anti-wrinkle agents, anti-inflammatory analgesics, antifungals, steroids, hair restorers, slimming agents, local anesthetics, antipruritics, antimicrobials, antivirals, keratin softeners, moisturizers, astringents, antioxidants, and hair growth inhibitors.

Item 2-13. A composition according to item 2-1, which is a liquid crystal composition for external use.

Item 2-14. A composition according to item 2-1, which is a microemulsion composition for external use.

Item 2-15. A composition according to item 2-1, which is a liposome composition for external use.

Item 2-16. A composition according to item 2-1, which is a pharmaceutical composition.

Item 2-17. A composition according to item 2-1, which is a cosmetic composition.

The present invention further provides the following compositions for external use.

Item 3-1. A composition for external use comprising (i) a phospholipid, (ii) a mono- or oligo-glycol ether, and (iv) at least one bioactive component selected from the group consisting of hyaluronic acid, hyaluronic acid derivatives, vitamin A, vitamin A derivatives, ascorbic acid, salts of ascorbic acid, dehydroascorbic acid, ascorbyl glucoside, ascorbyl stearate, L-ascorbyl dipalmitate, ascorbyl tetra-2-hexyldecanate, xanthine derivatives, ubiquinones, and salts thereof.

Item 3-2. A composition according to item 3-1, wherein the phospholipid (i) is at least one member selected from the group consisting of glycerophospholipids and sphingophospholipids.

Item 3-3. A composition according to item 3-1, wherein the phospholipid (i) is a hydrogenated phospholipid.

Item 3-4. A composition according to item 3-1, wherein the phospholipid (i) is a non-hydrogenated phospholipid.

Item 3-5. A composition according to item 3-1, wherein the phospholipid (i) is a lecithin.

Item 3-6. A composition according to item 2-5, wherein the lecithin has an iodine value of 10 to 50.

Item 3-7. A composition according to item 3-1, wherein the proportion of phospholipid (i) is 0.01 to 15 wt.% of the total amount of the composition.

Item 3-8. A composition according to item 3-1, wherein the mono- or oligo-glycol ether (ii) is a compound represented by Formula (1):

R₁-O- (A-O)ₙ-R₂ (1)

wherein R₁ and R₂ are the same or different, and each independently represent a C₁₋₆ alkyl group, a C₂₋₆ alkenyl group, an aryl group, an acetyl group, or a hydrogen atom, provided that at least one of R₁ and R₂ represents a C₁₋₆ alkyl group, a C₂₋₆ alkenyl group, or an aryl group;
A represents a C₂₋₄ alkylene group; and
n represents an integer of 1 to 4.

Item 3-9. A composition according to item 3-1, wherein the mono-or oligo-glycol ether (ii) is at least one member selected from the group consisting of ethylene glycol monoethers, ethylene glycol diethers, diethylene glycol monoethers, diethylene glycol diethers, triethylene glycol monoethers, triethylene glycol diethers, tetraethylene glycol monoethers, tetraethylene glycol diethers, propylene glycol diethers, dipropylene glycol monoethers, dipropylene glycol diethers, tripropylene glycol monoethers, tripropylene glycol diethers, butylene glycol monoethers, and butylene glycol diethers.

Item 3-10. A composition according to item 3-1, wherein the proportion of mono- or oligo-glycol ether (ii) is 0.01 to 80 wt.% of the total amount of the composition.

Item 3-11. A composition according to item 3-1, wherein the phospholipid (i) is a hydrogenated lecithin and/or a non-hydrogenated lecithin.

Item 3-12. A composition according to item 3-1, wherein the bioactive component (iv) is at least one member selected from the group consisting of hyaluronic acid, acetylhyaluronic acid, and salts thereof.

Item 3-13. A composition according to item 3-1, wherein the bioactive component (iv) is at least one member selected from the group consisting of retinol, retinol acetate, retinol palmitate, retinal, retinoic acid, methyl retinoate, ethyl retinoate, retinol retinoate, vitamin A fatty acid esters, d-δ-tocopheryl retinoate, α-tocopheryl retinoate, and β-tocopheryl retinoate.

Item 3-14. A composition according to item 3-1, wherein the bioactive component (iv) is at least one member selected from the group consisting of ascorbic acid, salts of ascorbic acid, dehydroascorbic acid, ascorbyl glucoside, ascorbyl stearate, L-ascorbyl dipalmitate, and ascorbyl tetra-2-hexyldecanoate.

Item 3-15. A composition according to item 3-1, wherein the bioactive component (iv) is at least one xanthine derivative selected from the group consisting of caffeine, aminophylline, theophylline, oxtriphylline, dyphylline, diisobutylaminobenzoyloxypropyl theophylline, theobromine, diprophylline, proxyphylline, and pentoxifylline.

Item 3-16. A composition according to item 3-1, wherein the bioactive component (iv) is at least one ubiquinone selected from the group consisting of coenzymes Q6, Q7, Q8, Q9, and Q10.

Item 3-17. A composition according to item 3-1, which is a liquid crystal composition for external use.

Item 3-18. A composition according to item 3-1, which is a microemulsion composition for external use.

Item 3-19. A composition according to item 3-1, which is a liposome composition for external use.

Item 3-20. A composition according to item 2-1, which is a pharmaceutical composition.

Item 3-21. A composition according to item 2-1, which is a cosmetic composition.

Furthermore, the present invention provides the following methods for improving percutaneous absorption of bioactive components.

Item 4. A method for improving percutaneous absorbability of (iii) a bioactive component, the method comprising incorporating (i) a phospholipid in a proportion of 0.01 to 8 wt.% of, and (ii) a mono- or oligo-glycol ether in a proportion of 0.01 to 6.5 wt.%, based on the total amount of a composition, into the composition containing the bioactive component (iii).

Item 5. A method for improving percutaneous absorbability of (iii) a bioactive component, the method comprising incorporating (i) a phospholipid in a proportion of 0.01 to 8 wt.%, and (ii) a mono- or oligo-glycol ether in a proportion of 0.01 to 6.5 wt.% of, based on a total amount of a composition, into the composition containing the bioactive component (iii).

Item 6. A method for improving percutaneous absorbability of (iv) at least one bioactive component selected from the group consisting of hyaluronic acid, hyaluronic acid derivatives, vitamin A, vitamin A derivatives, ascorbic acid, dehydroascorbic acid, ascorbyl glucoside, ascorbyl stearate, L-ascorbyl dipalmitate, ascorbyl tetra-2-hexyldecanoate, xanthine derivatives, ubiquinones, and salts thereof, the method comprising incorporating (i) a phospholipid and (ii) a mono- or oligo-glycol ether into a composition containing said least one bioactive component (iv).

The present invention further provides the following uses.

Item 7. Use of a composition for external use comprising (i) a phospholipid, and (ii) a mono- or oligo-glycol ether in a proportion of at least 22 wt.% of the total amount of the composition, for improving percutaneous absorption of (iii) a bioactive component.

Item 8. Use of a composition for external use comprising (i) a phospholipid, and (ii) a mono- or oligo-glycol ether in a proportion of at least 22 wt.% of the total amount of the composition, for the manufacture of a preparation in which percutaneous absorption of (iii) a bioactive component is improved.

Item 9. Use of a composition for external use comprising (i) a phospholipid in a proportion of 0.01 to 8 wt.%, (ii) a mono- or oligo-glycol ether in a proportion of 0.01 to 6.5 wt.%, based on the total amount of the composition, for promoting percutaneous absorption of (iii) a bioactive component.

Item 10. Use of a composition for external use comprising (i) a phospholipid in a proportion of 0.01 to 8 wt.%, and (ii) a mono- or oligo-glycol ether in a proportion of 0.01 to 6.5 wt.%, based on the total amount of the composition, for the manufacture of a preparation in which percutaneous absorption of (iii) a bioactive component is improved.

Item 11. Use of a composition for external use comprising (i) a phospholipid and (ii) a mono- or oligo-glycol ether, for improving percutaneous absorption of (iv) at least one bioactive component selected from the group consisting of hyaluronic acid, hyaluronic acid derivatives, vitamin A, vitamin A derivatives, ascorbic acid, dehydroascorbic acid, ascorbyl glucoside, ascorbyl stearate, L-ascorbyl dipalmitate, ascorbyl tetra-2-hexyldecanoate, xanthine derivatives, ubiquinones, and salts thereof.

Item 12. Use of a composition for external use comprising (i) a phospholipid and (ii) a mono- or oligo-glycol ether, for the manufacture of a preparation in which percutaneous absorption of (iv) at least one bioactive component selected from the group consisting of hyaluronic acid, hyaluronic acid derivatives, vitamin A, vitamin A derivatives, ascorbic acid, dehydroascorbic acid, ascorbyl glucoside, ascorbyl stearate, L-ascorbyl dipalmitate, ascorbyl tetra-2-hexyldecanoate, xanthine derivatives, ubiquinones, and salts thereof, is improved.

### BEST MODE FOR CARRYING OUT THE INVENTION

### A. Composition for External Use

The composition for external use of the present invention comprises (i) a phospholipid and (ii) a mono- or oligo-glycol ether. Phospholipids and mono- or oligo-glycol ethers for use in the present invention are described below.

### 1. Phospholipid

Usable phospholipids are not limited as long as their use in compositions for external use is acceptable. Specific examples of such phospholipids include glycerophospholipids, sphingophospholipids, etc.

Glycerophospholipids are lipids with glycerophosphate skeletons, and include those in which, as a lipophilic group, a long-chain fatty acid is linked via an ester bond, or a long-chain alcohol is linked via an ether bond, or a long-chain fatty acid is linked via a vinyl ether bond, to glycerol. Specific examples include phosphatidylcholine, phosphatidylethanolamine, phosphatidylserine, phosphatidylinositol, phosphatidylinositol polyphosphate, phosphatidylglycerol, diphosphatidylglycerol (cardiolipin), phosphatidic acid, lysophosphatidylcholine, lysophosphatidylethanolamine, lysophosphatidylserine, lysophosphatidylinositol, lysophosphatidylglycerol, lysophosphatidic acid, etc.

Sphingophospholipids are phospholipids in which, in a sphingosine or phytosphingosine, a long-chain fatty acid is linked via an amide bond to the amino group at the C-2 position, and phosphoric acid or phosphonic acid is linked via a phosphoric ester bond to the hydroxy group at the C-1 position. Specific examples of sphingophospholipids include sphingomyelin and like ceramide-1-phosphate derivatives; and ceramide aminoethylphosphonate and like ceramide-1-phosphonate derivatives.

Among these phospholipids, glycerophospholipids are preferable, with phosphatidylcholine, phosphatidylethanolamine, and phosphatidylglycerol being particularly preferable.

The phospholipid(s) for use in the present invention may be any of natural phospholipids that have been extracted and purified from animals or plants, and chemically synthesized phospholipids. Also usable are phospholipids processed by hydrogenation, hydroxylation, or other treatments. Commercially available phospholipids can also be used. Known natural phospholipids include lecithins, which are purified from soybeans, egg yolks, etc. In the present invention, lecithins can be particularly preferably used as phospholipids.

Lecithins may be non-hydrogenated lecithins, hydrogenated lecithins, hydroxylated lecithins, or the like. Non-hydrogenated lecithins and hydrogenated lecithins are preferable, and non-hydrogenated lecithins are particularly preferable. Among hydrogenated lecithins, partially hydrogenated lecithins are preferable, and the lower the hydrogenation degree, the more preferable. The iodine value of lecithins is usually 10 to 50, preferably 20 to 45, and more preferably 30 to 45.

### 2. Mono- or Oligo-Glycol Ether

Mono- or oligo-glycol ethers are compounds in which one or both of the hydroxy groups of mono- or oligo-alkyleneglycol have been etherified. In oligoglycol ethers, the oligoalkylene glycol moiety may comprise two or more kinds of alkylene glycols. Usable mono- or oligo-glycol ethers in the present invention are not limited as long as they can be used in compositions for external use, and preferable examples thereof include compounds represented by Formula (1).

R₁-O- (A-O)ₙ-R₂ (1)

In Formula (1), R₁ and R₂ are the same or different, and each independently represent a C₁₋₆ alkyl group, a C₂₋₆ alkenyl group, an aryl group, an acetyl group, or a hydrogen atom, provided that at least one of R₁ and R₂ represents a C₁₋₆ alkyl group, a C₂₋₆ alkenyl group, or an aryl group. The C₁₋₆ alkyl groups include straight- or branched-chain alkyl groups having 1 to 6 carbon atoms. Examples of such alkyl groups include methyl, ethyl, n-propyl, isopropyl, n-butyl, isobutyl, tert-butyl, sec-butyl, n-pentyl, neopentyl, n-hexyl, isohexyl, 3-methylpentyl, etc.

Specific examples of C₂₋₆ alkenyl groups include vinyl, allyl, hexadiene, etc.

Specific examples of aryl groups include phenyl, tolyl, xylyl, naphthyl, etc.

In Formula (1), "A" represents a C₂₋₄ alkylene group. Specific examples of C₂₋₄ alkylene groups include ethylene, trimethylene, tetramethylene, etc.

In Formula (1), "n" represents an integer of 1 to 4, which indicates the polymerization degree of glycol.

Examples of mono- or oligo-glycol ethers that can be used in the present invention include ethylene glycol monoethers, ethylene glycol diethers, diethylene glycol monoethers, diethylene glycol diethers, triethylene glycol monoethers, triethylene glycol diethers, tetraethylene glycol monoethers, tetraethylene glycol diethers, propylene glycol monoethers, propylene glycol diethers, dipropylene glycol monoethers, dipropylene glycol diethers, tripropylene glycol monoethers, tripropylene glycol diethers, butylene glycol monoethers, butylene glycol diethers, etc.

Specific examples of mono- or oligo-glycol ethers include ethylene glycol monovinyl ether, ethylene glycol monoethyl ether, ethylene glycol mono-n-propyl ether, ethylene glycol monoisopropyl ether, ethylene glycol mono-n-butyl ether, ethylene glycol monoisobutyl ether, ethylene glycol mono-t-butyl ether, ethylene glycol mono-2-methylpentyl ether, ethylenyl glycol mono-n-hexyl ether, ethylene glycol mono-2,4-hexadiene ether, ethylene glycol mono-2,6,8-trimethyl-4-nonyl ether, ethylene glycol monophenyl ether, ethylene glycol monotolyl ether, ethylene glycol monomethyl ether acetate, ethylene glycol monoethyl ether acetate, ethylene glycol mono-n-butyl ether acetate, and like ethylene glycol monoethers; ethylene glycol dimethyl ether, ethylene glycol diethyl ether, and like ethylene glycol diethers; diethylene glycol monomethyl ether, diethylene glycol monoethyl ether (hereinafter sometimes referred to as "ethoxy diglycol"), diethylene glycol mono-n-propyl ether, diethylene glycol mono-n-butyl ether, diethylene glycol monoisobutyl ether, diethylene glycol mono-n-hexyl ether, diethylene glycol monomethylphenyl ether, diethylene glycol monoethyl ether acetate, diethylene glycol mono-n-butyl ether acetate, and like diethylene glycol monoethers; diethylene glycol dimethyl ether, diethylene glycol divinyl ether, and like diethylene glycol diethers; triethylene glycol monomethyl ether, triethylene glycol monoethyl ether, triethylene glycol mono-n-butyl ether, triethylene glycol monovinylethyl ether, and like triethylene glycol monoethers; triethylene glycol dimethyl ether, triethylene glycol divinyl ether, and like triethylene glycol diethers; tetraethylene glycol monophenyl ether and like tetraethylene glycol monoethers; tetraethylene glycol diethyl ether and like tetraethylene glycol diethers; oligoethylene glycol monomethyl ether and like oligoethylene glycol monoethers; propylene glycol monomethyl ether, propylene glycol monoethyl ether, propylene glycol-mono-n-propyl ether, propylene glycol monoisopropyl ether, propylene glycol mono-n-butyl ether, propylene glycol monoisobutyl ether, propylene glycol monoallyl ether, propylene glycol monophenyl ether, propylene glycol monomethyl ether acetate, and like propylene glycol monoethers; propylene glycol dimethyl ether, propylene glycol diethyl ether, propylene glycol di-n-propyl ether, propylene glycol diisopropyl ether, propylene glycol di-n-butyl ether, propylene glycol diisobutyl ether, propylene glycol diallyl ether, propylene glycol diphenyl ether, and like propylene glycol diethers; dipropylene glycol monoethyl ether, dipropylene glycol mono-n-butyl ether, dipropylene glycol monoisobutyl ether, dipropylene glycol allyl ether, and like dipropylene glycol monoethers; dipropylene glycol diethyl ether, dipropylene glycol di-n-butyl ether, dipropylene glycol diisobutyl ether, dipropylene glycol allyl ether, and like dipropylene glycol diethers; tripropylene glycol monomethyl ether, tripropylene glycol monoethyl ether, tripropylene glycol mono-n-butyl ether, tripropylene glycol monoisobutyl ether, tripropyleneglycol monoallyl ether, and like tripropylene glycol monoethers; tripropylene glycol dimethyl ether, tripropylene glycol diethyl ether, tripropylene glycol din-butyl ether, tripropylene glycol diisobutyl ether, tripropylene glycol diallyl ether, and like tripropylene glycol diethers; oligopropylene glycol monobutyl ether, and like oligopropylene glycol monoethers; butylene glycol monomethyl ether, butylene glycol monoethyl ether, butylene glycol mono-n-butyl ether, and like butylene glycol monoethers; butylene glycol dimethyl ether, butylene glycol diethyl ether, butylene glycol di-n-butyl ether, and like butylene glycol diethers, etc.

In the present invention, preferable mono- or oligo-glycol ethers include compounds of Formula (1) in which A is a C₂ or C₃ alkylene group. Specific examples of such compounds include ethylene glycol monoethers, ethylene glycol diethers, diethylene glycol monoethers, diethylene glycol diethers, triethylene glycol monoethers, tetraethylene glycol monoethers, tetraethylene glycol diethers, oligoethylene glycol monoethers, propylene glycol monoethers, propylene glycol diethers, dipropylene glycol monoethers, dipropylene glycol diethers, and tripropylene glycol monoethers.

More specific examples of compounds of Formula (1) in which A is a C₂ or C₃ alkylene group include ethylene' glycol monovinyl ether, ethylene glycol monoethyl ether, ethylene glycol mono-n-propyl ether, ethylene glycol monoisopropyl ether, ethylene glycol mono-n-butyl ether, ethylene glycol monoisobutyl ether, ethylene glycol mono-t-butyl ether, ethylene glycol mono-2-methylpentyl ether, ethylene glycol dimethyl ether, ethylene glycol diethyl ether, diethylene glycol monomethyl ether, diethylene glycol monoethyl ether, diethylene glycol mono-n-propyl ether, diethylene glycol mono-n-butyl ether, diethylene glycol monoisobutyl ether, diethylene glycol mono-n-hexyl ether, diethylene glycol monomethylphenyl ether, diethylene glycol dimethyl ether, diethylene glycol divinyl ether, diethylene glycol ethylvinyl ether, triethylene glycol monomethyl ether, triethylene glycol monoethyl ether, triethylene glycol mono-n-butyl ether, triethylene glycol monovinylethyl ether, tetraethylene glycol monophenyl ether, tetraethylene glycol diethyl ether, oligoethylene glycol monomethyl ether, propylene glycol monomethyl ether, propylene glycol monoethyl ether, propylene glycol mono-n-propyl ether, propylene glycol monoisopropyl ether, propylene glycol mono-n-butyl ether, propylene glycol phenyl ether, propylene glycol monomethyl ether, propylene glycol dimethyl ether, propylene glycol diethyl ether, propylene glycol di-n-propyl ether, propylene glycol diisopropyl ether, propylene glycol di-n-butyl ether, propylene glycol diisobutyl ether, propylene glycol diallyl ether, propylene glycol diphenyl ether, dipropylene glycol monoethyl ether, dipropylene glycol mono-n-butyl ether, dipropylene glycol diethyl ether, dipropylene glycol di-n-butyl ether, dipropylene glycol diisobutyl ether, dipropylene glycol monoallyl ether, tripropylene glycol monomethyl ether, tripropylene glycol monoethyl ether, and tripropylene glycol mono-n-butyl ether.

More preferable mono- or oligo-glycol ethers in the present invention include compounds of Formula (1) in which one of R₁ and R₂ is a C₁₋₆ alkyl group and the other is a hydrogen atom; A is a C₂ or C₃ alkylene group; and n is 1 or 2. Specific examples of such compounds include ethylene glycol monoethers, diethylene glycol monoethers, propylene glycol monoethers, and dipropylene glycol monoethers.

Particularly preferable mono- or oligo-glycol ethers for use in the present invention include compounds of Formula (1) in which one of R₁ and R₂ is a C₁₋₃ alkyl group and the other is a hydrogen atom; A is a C₂ or C₃ alkylene group; and n is 1 or 2. Specific examples of such compounds include ethylene glycol monomethyl ether, ethylene glycol monoethyl ether, ethylene glycol monopropyl ether, diethylene glycol monomethyl ether, diethylene glycol monoethyl ether, diethylene glycol mono-n-propyl ether, propylene glycol monoethyl ether, propylene glycol mono-n-propyl ether, dipropylene glycol monoethyl ether, and dipropylene glycol mono-n-propyl ether. Among these, diethylene glycol monoethyl ether is most preferable.

### 3. Embodiments of Combinations of (i) Phospholipids, (ii) Mono-or Oligo-Glycol Ethers, and (iii) bioactive Components

According to the present invention, in the composition for external use comprising the phospholipid (i), mono- or oligo-glycol ether (ii), and bioactive component (iii), the percutaneous absorbability of bioactive component (iii) can be improved when (1) the proportion of mono- or oligo-glycol ether (ii) is at least 22 wt.%; (2) the proportion of phospholipid (i) is 0.01 to 8 wt.% and that of mono- or oligo-glycol ether (ii) is 0.01 to 6.5 wt.%; or (3) specific bioactive component (s) (iv) is selected. These three embodiments are separately described below in detail.

### (1) Embodiment 1

Embodiment 1 of the present invention provides a composition for external use comprising (i) a phospholipid and (ii) a mono- or oligo-glycol ether, in which the proportion of mono- or oligo-glycol ether (ii) is within a specific range, so that the percutaneous absorbability of the bioactive component (iii) contained in the composition is improved.

That is, the present invention provides a composition for external use (hereinafter referred to as Composition 1) comprising (i) a phospholipid and (ii) a mono- or oligo-glycol ether, wherein the proportion of mono- or oligo-glycol ether(s) (ii) is at least 22 wt.% of the total amount of the composition.

The proportion of phospholipid(s) (i) in Composition 1 is not limited, but is usually 0.01 to 15 wt.%, preferably 0.05 to 10 wt.%, and more preferably 0.1 to 8 wt.%, of the total amount of Composition 1.

The proportion of mono- or oligo-glycol ether(s) (ii) in Composition 1 is at least 22 wt.% of the total amount of Composition 1. The proportion of mono- or oligo-glycol ether(s) (ii) is preferably 25 to 80 wt.%, and more preferably 30 to 70 wt.%. Use of the mono- or oligo-glycol ether(s) (ii) in such a proportion enables more efficient percutaneous absorption of the bioactive component.

In Composition 1, the ratio of mono- or oligo-glycol ether(s) (ii) to phospholipid(s) (i) can be suitably selected within the range that satisfies the above proportions of phospholipid(s) (i) and mono- or oligo-glycol ether(s) (ii). Specifically, the ratio of mono- or oligo-glycol ether(s) (ii) to phospholipid(s) (i) is usually 1 to 300 parts by weight, preferably 2 to 100 parts by weight, and more preferably 3 to 50 parts by weight, of the former per part by weight of the latter.

Composition 1 comprises (iii) a bioactive component, in addition to the above phospholipid (i) and mono- or oligo-glycol ether (ii). The bioactive component (iii) in Composition 1 is not limited as long as it can be used in compositions for external use and exhibits desired physiological activities when applied percutaneously. Examples of usable bioactive components include vitamin compounds, skin-whitening agents, anti-wrinkle agents, anti-inflammatory analgesics, antifungals, steroids, hair restorers, slimming agents, local anesthetics, antipruritics, antimicrobials, antivirals, keratin softeners, moisturizers, astringents, antioxidants, hair growth inhibitors, etc. Among such components, preferable examples are vitamin compounds (in particular, vitamin A compounds and water-soluble vitamin C compounds), skin-whitening agents, anti-wrinkle agents, anti-inflammatory analgesics, antifungals, steroids, hair restorers, and slimming agents. More preferable examples are vitamin A compounds, water-soluble vitamin C compounds, anti-wrinkle agents, and slimming agents. Such bioactive components can be used singly or in combination.

The proportion of bioactive component(s) (iii) used in Composition 1 is suitably selected depending on the kind(s) of bioactive component(s), the form of Composition 1, etc. The total proportion of the bioactive component(s) (iii) is usually 0.0001 to 50 wt.%, preferably 0.0005 to 40 wt.%, and more preferably 0.001 to 30 wt.%, of the total amount of Composition 1.

More specific examples of the kinds and proportions of bioactive components (iii) are given below, but are not limitative.

Examples of vitamin compounds include retinol, retinol acetate, retinol palmitate, retinal, retinoic acid, methyl retinoate, ethyl retinoate, retinol retinoate, vitamin A fatty acid esters, d-δ-tocopheryl retinoate, α-tocopheryl retinoate, β-tocopheryl retinoate, and like vitamin A compounds; β-carotene, α-carotene, γ-carotene, δ-carotene, lycopene, zeaxanthin, cryptoxanthin, echinenone, and like provitamin A compounds; dl-α-tocopherol succinate, dl-α-tocopherol calcium succinate, and like vitamin E compounds; riboflavin, flavin mononucleotide, flavin adenine dinucleotide, riboflavin butyrate, riboflavin tetrabutylate, sodium riboflavin-5'-phosphate, riboflavin tetranicotinate, and like vitamin B2 compounds; methyl nicotinate, nicotinic acid, nicotinamide, and like nicotinic acid compounds; ascorbyl stearate, L-ascorbyl dipalmitate, ascorbyl tetraisopalmitate, ascorbic acid, sodium ascorbate, dehydroascorbic acid, sodium ascorbyl phosphate, magnesium ascorbyl phosphate, magnesium ascorbyl phosphate, sodium ascorbyl phosphate, ascorbyl glucoside, and like vitamin C compounds; methylhesperidin, ergocalciferol, cholecalciferol, and like vitamin D compounds; phylloquinone, farnoquinone, and like vitamin K compounds; γ-oryzanol, dibenzoyl thiamine, dibenzoyl thiamine hydrochloride, thiamine hydrochloride, thiamine cetyl hydrochloride, thiamine thiocyanate, thiamine lauryl hydrochloride, thiamine nitrate, thiamine monophosphate, lysine salt of thiamine, thiamine triphosphate, phosphoric acid salt of thiamine monophosphate, thiamine monophosphate, thiamine diphosphate, thiamine diphosphate hydrochloride, thiamine triphosphate, monophosphoric acid salt of thiamine triphosphate, and like vitamin B1 compounds; pyridoxine hydrochloride, pyridoxine acetate, pyridoxal hydrochloride, pyridoxal 5'-phosphate, pyridoxamine hydrochloride, and like vitamin B6 compounds; cyanocobalamin, hydroxocobalamin, deoxyadenosylcobalamin, and like vitamin B12 compounds; folic acid, pteroylglutamic acid, and like folic acid compounds; pantothenic acid, calcium pantothenate, pantothenyl alcohol (panthenol), D-pantesin, D-pantethine, coenzyme A, pantothenyl ethyl ether, and like pantothenic acid compounds; biotin, bioticin, and like biotin compounds; carnitine, ferulic acid, α-lipoic acid, orotic acid, and like vitamin-like factors; etc.

The proportion of vitamin compound(s) used in the present invention is not limited, and can be suitably selected considering the feel on the skin, and the effect of physiological activity. For example, the proportion of vitamin compound(s) is usually 0.1 to 30 wt.%, preferably 0.5 to 25 wt.%, and more preferably 1 to 20 wt.%, of the total amount of Composition 1.

Examples of skin-whitening agents include placentas; arbutin; cysteine; ellagic acid; kojic acid; phytic acid; rucinol; hydroquinone; components, extracts, and essential oils derived from plants such as iris, almond, aloe, ginkgo, oolong tea, rose fruit, scutellaria root, *Coptis Rhizome,* St. John's wort *(Hypericum erectum Thunb),* dead nettle, seaweed, pueraria root, chamomile, licorice, gardenia, *Sophorae Radix,* wheat, rice, rice germ, orizanol, rice bran, perilla, peony, *Cnidium Rhizome,* mulberry bark, soybeans, tea, terminalia, Japanese angelica, *Calendula officinalis,* hamamelis, safflower, moutan bark, coix seeds, Japanese angelica, Celtis *sinensis,* persimmon (Diospyros *kaki),* clove, etc.; and the like. Among such agents, arbutin, cysteine, and terminalia extracts are preferable.

The proportion of skin-whitening agent(s) used in the present invention is not limited, and can be suitably selected considering the feel on the skin, and the effect of physiological activity. For example, the proportion of skin-whitening agent(s) is usually 0.1 to 30 wt.%, preferably 0.5 to 25 wt.%, and more preferably 1 to 20 wt.%, of the total amount of Composition 1.

Examples of anti-wrinkle agents include ubiquinones such as coenzymes Q6 to Q10, kinetin, glycolic acid, argireline, acylated glucosamine, collagens, hyaluronic acid, aloe extracts, seaweed extracts, horse chestnut extracts, rosemary extracts, cornflower extracts, etc., among which coenzyme Q10 and kinetin are preferable.

The proportion of anti-wrinkle agent(s) used in the present invention is not limited, and can be suitably selected considering the feel on the skin, and the effect of physiological activity. For example, the proportion of anti-wrinkle agent(s) is usually 0.1 to 30 wt.%, preferably 0.5 to 25 wt,%, and more preferably 1 to 20 wt.%, of the total amount of Composition 1.

Examples of anti-inflammatory analgesics include indomethacin, felbinac, methyl salicylate, glycol salicylate, allantoin, allantoin derivatives, ibuprofen, ibuprofen piconol, bufexamac, butyl flufenamate, bendazac, piroxicam, ketoprofen, etc., among which indomethacin, felbinac, and methyl salicylate are preferable.

The proportion of anti-inflammatory analgesic(s) used in the present invention is not limited, and can be suitably selected considering the feel on the skin, and the effect of physiological activity. For example, the proportion of anti-inflammatory analgesic(s) is usually 0.1 to 30 wt.%, preferably 0.5 to 25 wt,%, and more preferably 1 to 20 wt.%, of the total amount of Composition 1.

Examples of antifungals include terbinafine hydrochloride, sulconazole nitrate, clotrimazole, isoconazole nitrate, cloconazole nitrate, miconazole nitrate, econazole nitrate, oxiconazole nitrate, bifonazole, tioconazole, ketoconazole, tolnaftate, tolciclate, liranaftate, ciclopirox olamine, exalamide, siccanin, undecylenic acid, zinc undecylenate, pyrrolnitrin, butenafine hydrochloride, amorolfine hydrochloride, neticonazole hydrochloride, etc., among which terbinafine hydrochloride and sulconazole nitrate are preferable.

The proportion of antifungal(s) used in the present invention is not limited, and can be suitably selected considering the feel on the skin, and the effect of physiological activity. For example, the proportion of antifungal(s) is usually 0.1 to 30 wt.%, preferably 0.5 to 25 wt.%, and more preferably 1 to 20 wt.%, of the total amount of Composition 1.

Usable steroids include, for example, dexamethasone valerate acetate, dexamethasone, dexamethasone propionate, dexamethasone acetate, dexamethasone valerate, prednisolone valerate acetate, hydrocortisone butyrate, hydrocortisone acetate, hydrocortisone, hydrocortisone butyrate propionate, cortisone acetate, prednisolone acetate, prednisolone, betamethasone, betamethasone valerate, betamethasone dipropionate, clobetasone butyrate, clobetasol propionate, diflorasone acetate, diflucortolone valerate, beclometasone propionate, flumetasone pivalate, triamcinolone acetonide, fluocinolone acetonide, fluocinonide, amcinonide, halcinonide, difluprednate, etc., among which hydrocortisone acetate, prednisolone valerate acetate, and clobetasone butyrate are preferable.

The proportion of steroid(s) used in the present invention is not limited, and can be suitably selected considering the feel on the skin, and the effect of physiological activity. For example, the proportion of steroid(s) is usually 0.1 to 30 wt.%, preferably 0.5 to 25 wt,%, and more preferably 1 to 20 wt.%, of the total amount of Composition 1.

Examples of hair restorers include procyanidin, dipotassium glycyrrhizinate, carpronium chloride, cepharanthin, menthol, hinokitiol, L-hydroxyproline, acetyl hydroxyproline, fucoidan, capsicum tincture, cepharanthin, Swertianin, swertianine, flavonosteroids, immunotect, minoxidil, FGF-10, *Isodon japonicus Hara* extracts (essences), *Swertia japonica* extracts (essences), *Laminaria angustata* extracts (essences), *Gynostemma pentaphyllum* extracts (essences), St. John's wort *(Hypericum erectum Thunb*) extracts (essences), gentian extracts (essences), sage extracts (essences), peppermint extracts (essences), hop extracts (essences), coix seed extracts (essences), persimmon leaf extracts (essences), *Rehmanniae Radix* extracts (essences), carrot extracts (essences), *Tilia miqueliana* extracts (essences), moutan bark extracts (essences), etc. Among these, procyanidin, *Swertia japonica* extracts (essences), and *Laminaria angustata* extracts (essences) are preferable.

The proportion of hair restorer(s) used in the present invention is not limited, and can be selected considering the feel on the skin, and the effect of physiological activity. For example, the proportion of hair restorer(s) is usually 0.1 to 30 wt.%, preferably 0.5 to 25 wt.%, and more preferably 1 to 20 wt.%, of the total amount of Composition 1.

Usable slimming agents include, for example, caffeine, aminophylline, theophylline, oxtriphylline, dyphylline, diisobutylaminobenzoyloxypropyl theophylline, theobromine, diprophylline, proxyphylline, pentoxifylline, and like xanthines; capsaicin; etc. Among these, caffeine and capsaicin are preferable.

The proportion of slimming agent(s) used in the present invention is not limited, and can be selected considering the feel on the skin, and the effect of physiological activity. For example, the proportion of slimming agent(s) is usually 0.1 to 30 wt.%, preferably 0.5 to 25 wt.%, and more preferably 1 to 20 wt.%, of the total amount of Composition 1.

Examples of local anesthetics, antipruritics, antimicrobials, antivirals, keratin softeners, moisturizers, astringents, antioxidants, and hair growth inhibitors, are as follows.

Local anesthetics: lidocaine, lidocaine hydrochloride, dibucaine, dibucaine hydrochloride, ethyl aminobenzoate, eucalyptus oil, eugenol, camphor, peppermint oil, etc.

Antipruritics: crotamiton, chlorpheniramine, chlorpheniramine maleate, diphenhydramine, diphenhydramine hydrochloride, diphenhydramine salicylate, salicylic acid, nonylic acid vanillylamide, mequitazine, camphor, thymol, eugenol, polyoxyethylene lauryl ether, comfrey extracts, perilla extracts, etc.

Antimicrobials: isopropylmethylphenol, chlorhexidine gluconate, chlorhexidine hydrochloride, benzalkonium chloride, benzethonium chloride, cetyltrimethylammonium bromide, dequalinium chloride, triclosan, trichlorocarbanilide, etc.

Antivirals: acyclovir, penciclovir, etc.

Keratin softeners: ethyl alcohol, isopropyl alcohol, propanol, butanol, polyethylene glycol, benzyl alcohol, phenylethyl alcohol, propylene carbonate, hexyldodecanol, allantoin, dimethylsulfoxide, dimethylacetamide, dimethylformamide, triethanolamine, diisopropyl adipate, ethyl laurylate, lanolin, fatty acid dialkylol amide, urea, sulfur, resorcin, phytic acid, lactic acid, lactates, sodium hydroxide, potassium hydroxide, etc.

Moisturizers: hyaluronic acid, hyaluronic acid derivatives (e.g., acetylhyaluronic acid), salts of hyaluronic acid, polyethylene glycol, diglycerol-trehalose, heparin-like substances, sodium chondroitin sulfate, collagen, elastin, keratin, chitin, chitosan, and like high-molecular compounds; glycine, aspartic acid, arginine, and like amino acids; sodium lactate, urea, sodium pyrrolidone carboxylate, and like natural moisturizing factors; chamomile extracts, aloe extracts, aloe vera extracts, hamamelis extracts, rosemary extracts, thyme extracts, tea extracts, perilla extracts, and like plant extracts; etc.

Astringents: citric acid, tartaric acid, lactic acid, aluminium chloride, aluminium sulfate, allantoin chlorohydroxyaluminum, allantoin dihydroxyaluminum, aluminum phenolsulfonate, zinc paraphenolsulfonate, zinc sulfate, zinc lactate, aluminum chlorohydroxide, etc.

Antioxidants: dibutylhydroxytoluene, butylhydroxyanisole, disodium ethylenediaminetetraacetate dihydrate (hereinafter sometimes referred to as sodium edetate), sorbic acid, sodium sulfite, etc.

Hair growth inhibitors: isoflavones, blackberry lily extracts, dokudami *(Houttuynia cordata)* extracts, orris root extracts, papain enzyme, etc.

The proportions of local anesthetic(s), antipruritic(s), antimicrobial(s), antiviral(s), keratin softener(s), moisturizer(s), astringent(s), antioxidant(s), and hair growth inhibitor(s) in Composition 1 are not limited as long as the effects of the present invention are not impaired. The proportions can be suitably selected so that the upper limit of the pharmacologically or cosmetically acceptable range is not exceeded. Specifically, the proportion of each kind of bioactive component is usually 0.001 to 20 wt.%, preferably 0.001 to 10 wt.%, and more preferably 0.001 to 5 wt.%, of the total amount of Composition 1.

### (2) Embodiment 2

Embodiment 2 of the present invention provides a composition for external use that comprises specific proportions of (i) a phospholipid and (ii) a mono- or oligo-glycol ether, so that the percutaneous absorbability of bioactive component(s) contained in the composition is improved.

That is, the present invention provides a composition for external use (hereinafter referred to as Composition 2) comprising (i) a phospholipid in a proportion of 0.01 to 8 wt.%, and (ii) a mono- or oligo-glycol ether in a proportion of 0.01 to 6.5 wt.%, based on the total amount of Composition 2.

The proportion of phospholipid (i) in Composition 2 is 0.01 to 8 wt.% of the total amount of Composition 3. The proportion of phospholipid (i) is preferably 0.1 to 7.5 wt.%, and more preferably 1 to 7 wt.%.

The proportion of mono- or oligo-glycol ether (ii) in Composition 2 is 0.01 to 6.5 wt.% of the total amount of Composition 2. The proportion of mono- or oligo-glycol ether (ii) is preferably 0.1 to 6 wt.%, and more preferably 1 to 5 wt.%.

Use of the phospholipid (i) and mono- or oligo-glycol ether (ii) in the proportions within the above ranges enables more efficient percutaneous absorption of bioactive component(s).

In Composition 2, the ratio of mono- or oligo-glycol ether (ii) to phospholipid (i) is suitably selected within the range that satisfies the proportions of phospholipid (i) and mono- or oligo-glycol ether (ii) described above. Specifically, for example, the ratio of mono- or oligo-glycol ether (ii) to phospholipid (i) is usually 0.01 to 70 parts by weight, preferably 0.05 to 50 parts by weight, and more preferably 0.1 to 20 parts by weight, of the former per part by weight of the latter.

Composition 2 comprises, in addition to the phospholipid (i) and mono- or oligo-glycol ether (ii), bioactive component(s) (iii). The kind(s) and proportion(s) of bioactive component(s) (iii) used in Composition 2 are the same as those used in Composition 1 described above.

### (3) Embodiment 3

Embodiment 3 of the present invention provides a composition for external use in which the percutaneous absorbability of hyaluronic acid, hyaluronic acid derivative(s), vitamin A, vitamin A derivative(s), vitamin C, specific vitamin C derivative(s), xanthine derivative(s), ubiquinone(s), and/or salt(s) thereof is improved. The percutaneous absorbability of these specific bioactive components is improved by incorporating (i) a phospholipid and (ii) a mono- or oligo-glycol ether into a composition for external use.

That is, the present invention provides a composition for external use (hereinafter referred to as Composition 3) comprising (i) a phospholipid, (ii) a mono- or oligo-glycol ether, and (iv) at least one bioactive component selected from the group consisting of hyaluronic acid, vitamin A, vitamin A derivatives, vitamin C, specific vitamin C derivatives, xanthine derivatives, ubiquinones, and salts thereof.

The proportion of phospholipid (i) in Composition 3 is not limited, but is usually 0.01 to 15 wt.%, preferably 0.05 to 10 wt.%, and more preferably 0.1 to 8 wt.%, of the total amount of Composition 3.

The proportion of mono- or oligo-glycol ether (ii) in Composition 3 is also not limited, and can be suitably selected considering the feel on the skin, the percutaneous absorbability of bioactive components, etc. For example, the proportion is 0.01 to 80 wt.%, preferably 1 to 75 wt.%, and more preferably 5 to 70 wt.%, of the total amount of Composition 3.

In Composition 3 of the present invention, the ratio of mono- or oligo-glycol ether (ii) to phospholipid (i) is suitably selected within the range that satisfies the proportions of phospholipid (i) and mono- or oligo-glycol ether (ii) described above. Specifically, for example, the ratio of mono- or oligo-glycol ether (ii) to phospholipid (i) is usually 0.01 to 300 parts by weight, preferably 0.05 to 100 parts by weight, and more preferably 0.1 to 20 parts by weight, of the former per part by weight of the latter.

Composition 3 of the present invention comprises, as the bioactive component(s) (iv), one or more components selected from the group consisting of (iv-1) hyaluronic acid, hyaluronic acid derivatives, and/or salts thereof, (iv-2) vitamin A and/or vitamin A derivatives, (iv-3) vitamin C, salts thereof, and/or specific vitamin C derivatives, (iv-4) xanthine derivatives, and (iv-5) ubiquinones.

The proportion of bioactive component(s) (iv) in Composition 3 is suitably selected depending on the kind(s) of the bioactive component(s), form of Composition 3, etc., but the total proportion of bioactive component(s) (iv) is usually 0.0001 to 50 wt.%, preferably 0.0005 to 40 wt.%, and more preferably 0.001 to 30 wt.%, of the total amount of Composition 1.

The sources of (iv-1) hyaluronic acid, hyaluronic acid derivative(s), and/or salt(s) thereof (hereinafter sometimes referred to simply as "component (iv-1)") used in the present invention are not limited, and may be, for example, rooster combs or microorganisms.

Examples of hyaluronic acid derivatives include ester derivatives in which hydrogen atoms of hydroxy groups of hyaluronic acid are substituted with acyl groups. Examples of such ester derivatives include those having a total of 2 to 4 acyl groups per repeating unit (disaccharide) of hyaluronic acid. Specifically, such acyl groups include, for example, alkanoyl groups and arylcarbonyl groups. Preferable alkanoyl groups include those having 1 to 12 carbon atoms. Specific examples include acetyl, propionyl, butyryl, etc. Preferable arylcarbonyl groups include those having 7 to 15 carbon atoms. Specific examples include benzoyl, naphthylcarboxy, etc. Such alkanoyl groups and aryl carbonyl groups may be substituted with C₁₋₆ alkyl groups, C₁₋₆ alkoxy groups, halogen atoms, amino groups, hydroxy groups, and/or the like. When the alkanoyl groups and arylcarbonyl groups are substituted, the number of substituents are not limited, but is usually 1 to 3.

Other examples of hyaluronic acid derivatives include ether derivatives in which hydroxy groups of hyaluronic acid is substituted with alkoxy groups, aryloxy groups, and/or alkenyloxy groups. Examples of such ester derivatives include those in which the total number of alkoxy groups, aryloxy groups, and/or alkenyloxy groups is 1 to 3 per repeating unit (disaccharide) of hyaluronic acid. Preferable alkoxy groups include those having 1 to 6 carbon atoms, and specific examples include methoxy, ethoxy, n-propoxy, isopropoxy, n-butoxy, isobutoxy, t-butoxy, sec-butoxy, n-pentoxy, neopentoxy, n-hexyloxy, etc. Specific examples of aryloxy groups include phenyl, naphthyl, etc. Preferable alkenyloxy groups are those having 2 to 6 carbon atoms, and examples thereof include vinyloxy, allyloxy, etc. Such alkoxy groups, aryloxy groups, and/or alkenyloxy groups may be substituted with C₁₋₆ alkyl groups, C₁₋₆ alkoxy groups, halogen atoms, amino groups, hydroxy groups, and/or the like. When the alkoxy groups, aryloxy groups, and/or alkenyloxy groups are substituted, the number of substituents are not limited, but is usually 1 to 3.

Such derivatives of hyaluronic acid may be used singly or in combination with two or more of them. Preferable examples of hyaluronic acid derivatives include, for example, acetylhyaluronic acid, in which hydrogen atoms of hydroxy groups of hyaluronic acid are substituted with acetyl groups. Preferable examples of acetylhyaluronic acid are those having 2 to 4 acetyl groups as substituents per repeating unit (disaccharide) of hyaluronic acid.

As to the salts of hyaluronic acid and hyaluronic acid derivatives, a wide variety of pharmacologically or physiologically acceptable salts can be used. Examples of such salts include salts of alkali metals such as sodium, potassium, etc.; salts of alkaline earth metals such as magnesium, calcium, etc.; ammonium salts; salts of alkanolamines such as monoethanolamine and the like; etc.

The average molecular weight of hyaluronic acid and/or salts thereof is not limited, and is usually 1000 to 5000000, preferably 2000 to 4000000, and more preferably 3000 to 250000. It is known that due to such a high molecular weight, hyaluronic acid is not readily absorbed percutaneously and remains in the epidermis, even when it is incorporated into a conventional composition for external use and applied to the skin. Thus, conventional hyaluronic acid-containing compositions for external use, even when applied to the skin, do not exhibit the desired physiological activity of hyaluronic acid. In contrast, in Composition 3 of the present invention, hyaluronic acid overcomes such a defect and sufficiently exhibits its intrinsic physiological activity.

Preferable examples of component (iv-1) include alkali metal salts of hyaluronic acid, with sodium salt of hyaluronic acid being particularly preferable.

When the component (iv-1) is used as the bioactive component (iv) in Composition 3 of the present invention, the proportion thereof is suitably selected within the range of the proportion of bioactive component (iv) described above. A more suitable proportion of component (iv-1) is 0.0001 to 10 wt.%, preferably 0.0005 to 8 wt.%, and more preferably 0.001 to 5 wt.%, of the total amount of Composition 3.

Specific examples of (iv-2) vitamin A and/or vitamin A derivative(s) (hereinafter sometimes referred to simply as "component (iv-2)") used in the present invention include retinol, retinal, retinol acetate, retinoic acid, methyl retinoate, ethyl retinoate, retinol retinoate, vitamin A fatty acid esters, d-δ-tocopheryl retinoate, α-tocopheryl retinoate, β-tocopheryl retinoate, etc. Vitamin A fatty acid esters are compounds in which vitamin A is bound to C₁₂₋₁₈ fatty acids via ester bond. Specific examples thereof include retinol myristate, retinol stearate, retinol palmitate, etc. Such examples of component (iv-2) can be used singly or in combination with two or more of them. Vitamin A oil can also be used as the component (iv-2). Preferable examples of the component (iv-2) include d-δ-tocopheryl retinoate.

When the component (iv-2) is used as the bioactive component (iv) in Composition 3, the proportion thereof is suitably selected within the range of the proportion of bioactive component (iv) described above, based on the form of Composition 3, feel on the skin, desired physiological effects, etc. A more suitable proportion of component (iv-2) is 0.01 to 5 wt.%, preferably 0.1 to 3 wt.%, and more preferably 0.2 to 1 wt.%, of the total amount of Composition 3.

Specific examples of (iv-3) vitamin C, salt(s) thereof, and/or specific vitamin C derivative(s) (hereafter sometimes referred to simply as "component (iv-3)") include ascorbic acid, salts of ascorbic acid, dehydroascorbic acid, ascorbyl glucoside, ascorbyl stearate, L-ascorbyl dipalmitate, and/or ascorbyl tetra-2-hexyldecanoate (ascorbyl tetraisopalmitate). Examples of salts of ascorbic acid include alkali metal salts of ascorbic acid, and more specific examples include sodium ascorbate. Such examples of the component (iv-3) can be used singly or in combination with two or more of them. Preferable examples of the component (iv-3) include ascorbic acid, ascorbyl glucoside, and ascorbyl tetra-2-hexyldecanoate.

When the component (iv-3) is used as the bioactive component (iv) in Composition 3, the proportion thereof is suitably selected within the range of the proportion of bioactive component (iv) described above, based on the form of Composition 3, feel on the skin, desired physiological effects, etc. A more suitable proportion of component (iv-3) is 0.01 to 50 wt.%, preferably 0.05 to 40 wt.%, and more preferably 0.1 to 30 wt.%, of the total amount of Composition 3.

Specific examples of (iv-4) xanthine derivative(s) (hereafter sometimes referred to simply as "component (iv-4)") used in the present invention include caffeine, aminophylline, theophylline, oxtriphylline, dyphylline, diisobutylaminobenzoyloxypropyl theophylline, theobromine, diprophylline, proxyphylline, pentoxifylline, etc. Such examples of the component (iv-4) can be used singly or in combination with two or more of them. Preferable examples of the component (iv-4) include caffeine, aminophylline, theophylline, diprophylline, proxyphylline, and theobromine, among which caffeine is particularly preferable.

When the component (iv-4) is used as the bioactive component (iv) in Composition 3, the proportion thereof is suitably selected within the range of he proportion of bioactive component (iv) described above, based on the form of Composition 3, feel on the skin, desired physiological effects, etc. A more suitable proportion of component (iv-4) is 0.01 to 50 wt.%, preferably 0.1 to 3 wt.%, and more preferably 0.2 to 1 wt.%, of the total amount of Composition 3.

(iv-5) Ubiquinones (hereinafter sometimes referred to simply as "component (iv-5)") used in the present invention are a general term for 2,3-dimethoxy-5-methyl-6-polyprenyl-1,4-benzoquinone, which is also called coenzyme Q. Usable ubiquinones include those having, in the side chain, usually 1 to 20 isoprene units, preferably 5 to 15 isoprene units, more preferably 6 to 10 isoprene units (coenzymes Q6 to Q10), and even more preferably 10 isoprene units (coenzyme Q10). Such examples of the component (iv-5) can be used singly or in combination with two or more of them.

When the component (iv-5) is used as the bioactive component (iv) in Composition 3, the proportion thereof is suitably selected within the range of the proportion of bioactive component (iv) described above, based on the form of Composition 3, feel on the skin, desired physiological effects, etc. A more suitable proportion of component (iv-5) is 0.001 to 5 wt.%, preferably 0.05 to 3 wt.%, and more preferably 0.01 to 1 wt.%, of the total amount of Composition 3.

Since Composition 3 also promotes the percutaneous absorption of components other than the components (iv-1) to (iv-5) described above, other bioactive components can be used to impart useful physiological activities.

The kinds of usable bioactive components other than the components (iv-1) to (iv-5) are not limited as long as they exhibit beneficial effects on the skin. Specific examples of other bioactive components that can be used in Composition 3 are components other than the components (iv-1) to (iv-5) among those listed as the bioactive components (iii) for use in Composition 1. The proportions of such other bioactive components are the same as in Composition 1.

In the following "4. Other Components", "5. Form of the Composition for External Use", and "6. Use", the term "composition for external use" encompasses all of Compositions 1 to 3 described above, unless otherwise indicated.

### 4. Other Components

The composition for external use of the present invention may further comprise water, if necessary. When water is contained in the composition for external use of the present invention, the proportion thereof is not limited, but is usually 0.001 to 75 wt.%, preferably 0.01 to 70 wt.%, and more preferably 0.1 to 65 wt.%, of the total amount of the composition.

The composition for external use of the present invention may further comprise polyhydric alcohol(s). When the composition contains polyhydric alcohol(s), the composition, in some cases, exhibits an advantageous effect of further promoting the percutaneous absorption of the bioactive components. Polyhydric alcohols that are generally used in compositions for external use can be used without limitation, and examples of usable polyhydric alcohols include glycerol, ethylene glycol, propylene glycol, trimethylene glycol, 1,2-butylene glycol, 1,3-butylene glycol, 2,3-butylene glycol, isobutylene glycol, 1,2-pentylene glycol, 1,2-hexylene glycol, octylene glycol, condensation products thereof, etc. Examples of condensation products include diglycerol, diethylene glycol, triethylene glycol, tetraethylene glycol, dipropylene glycol, tripropylene glycol, etc. When polyhydric alcohol(s) is contained in the composition for external use of the present invention, the proportion thereof is not limited, but is usually 1 to 40 wt.%, preferably 5 to 35 wt.%, and more preferably 10 to 30 wt.%, of the total amount of the composition.

The composition for external use of the present invention may contain, in addition to the above components, various base materials, carriers, other additives, and other components that are generally used in the field of medicines, quasi drugs, or cosmetics, within a range that does not deteriorate qualities such as storage stability, viscosity, etc., and that does not impair the effects of the present invention. Such components include, for example, base materials, surfactants, thickeners, preservatives, pH adjusters, stabilizers, irritation-reducing agents, antiseptics, coloring agents, dispersing agents, perfumes, etc. Such components can be used singly or in combination with two or more of them. Specific examples of such components include, but are not limited to, the following.

Base materials: paraffin, gelled hydrocarbons, ozokerite, ceresin, petrolatum, hard fats, microcrystalline waxes, and like hydrocarbons; lauric acid, myristic acid, palmitic acid, stearic acid, behenic acid, isostearic acid, oleic acid, linoleic acid, and like fatty acids; glyceryl tri-2-ethylhexanoate (trioctanoin), and like trifatty acid glycerides; highly polymerized methylpolysiloxane, dimethylsiloxane-methyl(polyoxyethylene)siloxane-methyl(polyoxypropylene)siloxane copolymers, dimethylsiloxane-methyl(polyoxyethylene)siloxane copolymers, dimethylsiloxane-methyl(polyoxypropylene)siloxane copolymers, polyoxyethylene-methylpolysiloxane copolymers, poly(oxyethylene-oxypropylene)-methylpolysiloxane copolymers, dimethylsiloxane-methylcetyloxysiloxane copolymers, dimethylsiloxane-methylstearoxysiloxane copolymers, alkyl acrylate copolymer methylpolysiloxane esters, crosslinked methylpolysiloxanes, crosslinked methylphenylpolysiloxanes, crosslinked polyether-modified silicones, crosslinked alkyl polyether-modified silicones, crosslinked alkyl-modified silicones, and like polymerized silicones; ethylene glycol monoacetate, ethylene glycol diacetate, triethylene glycol diacetate, hexylene glycol diacetate, 2-methyl-2-propene-1,1-diol diacetate, and like glycol acetates; triethylene glycol divalerate, 2,2,4-trimethyl-1,3-pentanediol monoisobutyrate, 2,2,4-trimethyl-1,3-pentanediol diisobutyrate, and like glycol esters; ethylene glycol diacrylate, diethylene glycol diacrylate, propylene glycol monoacrylate, 2,2-dimethyl-trimethylene glycol diacrylate, 1,3-butylene glycol diacrylate, and like glycol acrylates; ethylene glycol dinitrate, diethylene glycol dinitrate, triethylene glycol dinitrate, propylene glycol dinitrate, and like glycol dinitrates; 2,2'-[1,4-phenylenedioxy]diethanol; dioxanes; polyester of butylene glycol adipate; etc.

Surfactants: sorbitan monoisostearate, sorbitan monolaurate, sorbitan monopalmitate, sorbitan monostearate, diglycerol sorbitan penta-2-ethylhexylate, diglycerol sorbitan tetra-2-ethylhexylate, and like sorbitan fatty acid esters; glyceryl monostearate, glyceryl monostearate malate, and like glyceryl fatty acids; polyglyceryl monoisostearate, polyglyceryl diisostearate, and like polyglyceryl fatty acids; propylene glycol monostearate, and like propylene glycol fatty acid esters; polyoxyethylene hydrogenated castor oil 40, polyoxyethylene hydrogenated castor oil 60, polyoxyethylene hydrogenated castor oil 80, and like hydrogenated castor oil derivatives; polyoxyethylene (20) sorbitan monolaurate (polysorbate 20), polyoxyethylene (20) sorbitan monostearate (polysorbate 60), polyoxyethylene (20) sorbitan monooleate (polysorbate 80), and like polyoxyethylene sorbitan fatty acid esters; polyoxyethylene glyceryl monococoate, glycerol alkyl ethers, alkyl glucosides, polyoxyethylene cetyl ethers, stearylamine, oleylamine, etc.

Thickeners: guar gum, locust bean gum, carrageenan, xanthan gum, dextran, methylcellulose, ethylcellulose, carboxymethylcellulose, hydroxyethylcellulose, hydroxyethylcellulose, hydroxypropylcellulose, hydroxypropylmethylcellulose, sodium alginate, propylene glycol alginate, polyvinyl alcohols, polyvinylpyrrolidones, polyvinyl methyl ethers, carboxyvinyl polymers, acrylic acid-alkyl methacrylate copolymers, sodium polyacrylates, polyethylene glycols, bentonite, dextrin fatty acid esters, pectin, etc.

Preservatives: benzoic acid, sodium benzoate, dehydroacetic acid, sodium dehydroacetate, isobutyl paraoxybenzoate, isopropyl paraoxybenzoate, butyl paraoxybenzoate, ethyl paraoxybenzoate, propyl paraoxybenzoate, benzyl paraoxybenzoate, methyl paraoxybenzoate, phenoxyethanol, etc.

pH Adjusters: hydrochloric acid, sulfuric acid, phosphoric acid, polyphosphoric acid, boric acid and like inorganic acids; lactic acid, acetic acid, citric acid, tartaric acid, malic acid, succinic acid, sodium succinate, oxalic acid, gluconic acid, fumaric acid, propionic acid, acetic acid, aspartic acid, epsilon-aminocaproic acid, glutamic acid, aminoethylsulfonic acid, and like organic acids; gluconolactones; ammonium acetate; sodium bicarbonate, sodium carbonate, potassium hydroxide, sodium hydroxide, calcium hydroxide, magnesium hydroxide, and like inorganic bases; monoethanolamine, triethanolamine, diisopropanolamine, tri-isopropanolamine, lysine, and like organic bases; etc.

The proportions of such components are not limited as long as they do not impair the effects of the invention. Specifically, the proportion of each of such components is usually 0.001 to 20 wt.%, preferably 0.001 to 10 wt.%, and more preferably 0.001 to 5 wt.%, of the total amount of the composition for external use.

### 5. Form of Composition for External Use

The composition for external use of the present invention may be in the form of liquid, semi-solid, or solid. The composition of the present invention can therefore be formulated into various forms depending on the intended use. The form of the composition of the present invention may be, for example, an ointment, cream preparation, liquid preparation, gel preparation, solid preparation, patch, etc. The liquid preparation encompasses oils, lotions, milky lotions, aerosols, liquid crystals, microemulsions, and liposomes. The cream preparation and gel preparation encompass liquid crystals, microemulsions, and liposomes. The patch encompasses packs, liquid crystals, microemulsions, and liposomes. The solid preparation encompasses stick-shaped preparations. Among these, liquid preparations and gel preparations are preferable, with gel preparations being particularly preferable. The definitions, specific embodiments, etc., of "liquid crystal", "microemulsion" and "liposome" are described below.

Liquid crystals are liquids that generally have high crystallinity with a long orientation in the constituent molecules and have intermediate fluidity and viscosity between solid and liquid. Many liquid crystals are optically anisotropic. Liquid crystals form hexagonal layers or lamella layers, and such a structure produces color stripes or causes refraction or reflection of polarized light, when irradiated with white light. Thus, the formation of liquid crystals can be easily detected by the naked eye or by microscopic observation of multi-refraction. Since liquid crystals are analogous in structure to biological bilayers and intercellular lipids, they have the advantages of a high compatibility with the skin and a capability of promoting the percutaneous absorption of useful components. For such reasons, a liquid crystal composition in which liquid crystals are dispersed is a preferable form of the composition for external use of the present invention. Production processes for liquid crystal compositions for external use are known. For example, the liquid crystal composition can be prepared by admixing phospholipid(s), a water-soluble compound(s), a small amount of water, etc., to mono- or oligo-glycol ether(s), and further adding oil(s), oil-soluble compound(s), etc., as required. The liquid crystal composition may comprise a small amount of water, but may also be formulated as a substantially anhydrous preparation, without adding water.

Microemulsions are advantageous in that they can be more readily absorbed through the stratum corneum into the skin, than ordinary emulsions, since the micelles dispersed in microemulsions are small. For such reasons, a microemulsion composition comprising a microemulsion is a preferable form of the composition for external use of the present invention. The microemulsion preferably has a mean particle diameter of not more than 0.5 µm, in order to ensure optical transparency. Production processes for microemulsion compositions for external use are known. For example, the microemulsion composition can be prepared by admixing phospholipid(s) and other component(s) to mono- or oligo-glycol ether(s), then admixing oil(s), oil-soluble compound(s), etc., as required, and further adding water-soluble compound(s) and water as required, followed by emulsification using a high pressure homomixer.

Liposomes are vesicles consisting of spherical lipid bilayers. Since liposomes are analogous in structure to biological bilayers, they have the advantages of a high compatibility with the skin and a capability of promoting the percutaneous absorption of bioactive components. For such reasons, a liposome composition comprising liposomes is a preferable form of the composition for external use of the present invention. The liposomes may be single-layered or multi-layered. Production processes for liposome compositions for external use are known. For example, the liposome composition can be prepared by suitably admixing phospholipid(s), water-soluble compound(s), water, etc., to mono- or oligo-glycol ether(s), and further suitably admixing oil(s), oil-soluble compound(s), etc.

### 6. Use

The composition for external use of the present invention may be a pharmaceutical (including quasi drugs) or cosmetic, and finds various applications depending on the kind(s) of bioactive component(s) contained in the composition.

The amount and method of application of the composition for external use of the present invention to the skin can be suitably selected according to the kind(s) and proportion(s) of bioactive component(s), the form of the composition, and the like.

The composition for external use of the present invention can be prepared by incorporating the components in a standard manner according to the form of the composition.

### B. Method for Improving Percutaneous Absorption

As described above in "A. Composition for External Use", "3. (1) Embodiment 1", the present invention makes it possible to improve the percutaneous absorbability of (iii) a bioactive component by incorporating (i) a phospholipid and (ii) a mono- or oligo-glycol ether into a composition for external use containing the bioactive component (iii) so that the proportion of mono-or oligo-glycol ether (ii) becomes at least 22 wt.% of the composition. That is, the present invention also provides a method for improving percutaneous absorbability of (iii) a bioactive component, the method comprising incorporating (i) a phospholipid in a proportion of 0.01 to 8 wt.%, and (ii) a mono-or oligo-glycol ether in a proportion of 0.01 to 6.5 wt.% of, based on a total amount of a composition, into the composition containing the bioactive component (iii).

Further, as described above in "A. Composition for External Use", "3. (2) Embodiment 2", the present invention makes it possible to improve the percutaneous absorbability of (iii) a bioactive component by incorporating (i) a phospholipid and (ii) a mono- or oligo-glycol ether into a composition for external use containing the bioactive component (iii) so that the proportions of components (i) and (ii) become 0.01 to 8 wt.% and 0.01 to 6.5 wt.%, respectively. That is, the present invention also provides a method for improving percutaneous absorbability of (iii) a bioactive component, the method comprising incorporating (i) a phospholipid in a proportion of 0.01 to 8 wt.%, and (ii) a mono-or oligo-glycol ether in a proportion of 0.01 to 6.5 wt.% of, based on a total amount of a composition, into the composition containing the bioactive component (iii).

Furthermore, as described above in "A. Composition for External Use", "3. (3) Embodiment 3", the present invention makes it possible to improve the percutaneous absorbability of (iv) a specific bioactive component by incorporating (i) a phospholipid and (ii) a mono- or oligo-glycolether into a composition for external use containing the specific bioactive component (iv). That is, the present invention also provides a method for improving the percutaneous absorbability of (iv) at least one bioactive component selected from the group consisting of hyaluronic acid, vitamin A, vitamin A derivatives, vitamin C, specific vitamin C derivatives, xanthine derivatives, ubiquinones, and salts thereof, the method comprising incorporating the phospholipid (i) and mono- or oligo-glycol ether (ii) into a composition for external use containing said at least one bioactive component (iv).

### EXAMPLES

The following Examples describe the present invention in more detail, but are not intended to limit the scope of the invention. In the Examples, the proportions are expressed in percent by weight unless otherwise indicated.

### Test Example 1-1

Compositions for external use were prepared according to the formulations shown in Table 1. Specifically, predetermined amounts of phospholipid, oligoglycol ether, and polyhydric alcohol were heated to obtain a solution, and an aqueous solution of ascorbic acid that had been separately heated was mixed with the resulting solution. Predetermined amounts of other necessary components were added to the above-obtained mixture, to prepare the compositions for external use of Examples 1-1 and 1-2 and Comparative Examples 1-1 to 1-3, all of which are liposome compositions, except for the composition of Comparative Example 1-3. Each of the compositions was tested as follows. Ten milliliters of phosphate buffer (pH 7.4) was added to the reservoir compartment of a vertical Franz cell, and full-thickness skin from a hairless mouse (HR-1 strain, 7-week-old, male), from which the fat had been removed, was fixed between the cells. Thereafter, 1 ml of the composition was added to the donor compartment. Thirty hours after adding the composition, the ascorbic acid concentration in the phosphate buffer in the reservoir compartment was determined by HPLC.

Table 1 shows the results. In Table 1, the permeated amount (µg/cm²) means the amount (µg) of ascorbic acid that permeated during the 30 hours that followed the addition of the composition, per cm² of the hairless mouse full-thickness skin.

**Table 1**

| g/100 g | Ex.1-1 | Ex.1-2 | Comp.Ex. 1-1 | Comp.Ex. 1-2 | Comp.Ex. 1-3 |
|---|---|---|---|---|---|
| Hydrogenated soybean phospholipid *1 | 5 | - | 5 | 5 | - |
| Soybean phospholipid *2 | - | 1 | 5 | 5 | - |
| Ascorbic acid | 10 | 20 | 10 | 10 | 20 |
| Ethoxy diglycol | 43 | 49 | - | - | 53 |
| Glycerol | - | - | 43 | - | - |
| 1,3-Butylene glycol | - | - | - | 43 | - |
| Oleylamine | 1 | 1 | - | - | - |
| Diglycerol | - | - | - | - | 4 |
| Trimethylglycine | - | - | - | - | 3 |
| Purified water | Balance | Balance | Balance | Balance | Balance |
| Permeated amount (30 hours) µg/cm² | 1118 | 6350 | 359 | 397 | 174 |

| | | | | | |
|---|---|---|---|---|---|
| *1 SLP-PC92H (iodine value: not more than 45); product of Tsuji Oil Mill Co., Ltd. *2 SLP-PC70; product of Tsuji Oil Mill Co., Ltd. | | | | | |

When using the composition of Example 1-1, the permeated amount of ascorbic acid was 2.8 times or more that obtained when using the composition of Comparative Examples 1-1 or 1-2. Further, when using the composition of Example 1-1, the permeated amount of ascorbic acid was 6.4 times or more that obtained when using the composition of Comparative Example 1-3. Furthermore, when using the composition of Example 1-2, which contained non-hydrogenated soybean phospholipid, the permeated amount of ascorbic acid was about 16 times or more that obtained when using the compositions of Comparative Example 1-1 and 1-2, and 36 times or more that obtained when using the composition of Comparative Example 1-3.

### Test Example 1-2

Compositions for external use were prepared according to the formulations shown in Table 2. Specifically, predetermined amounts of phospholipid, oligoglycol ether, and polyhydric alcohol were heated to obtain a solution, which was then mixed with an aqueous solution of ascorbic acid and oleylamine that had been separately heated, to prepare the compositions of Examples 1-3 to 1-5.

When using any of the compositions of Example 1-3 to 1-5, the percutaneous absorbability of ascorbic acid was excellent.

**Table 2**

| g/100 g | Ex.1-3 | Ex.1-4 | Ex.1-5 |
|---|---|---|---|
| Hydrogenated soybean phospholipid * | 5 | 1 | 10 |
| Ascorbic acid | 10 | 10 | 5 |
| Ethoxy diglycol | 43 | 43 | 25 |
| Oleylamine | 1 | 0.5 | 1 |
| 1,3-Butylene glycol | - | - | 18 |
| Purified water | Balance | Balance | Balance |

| | | | |
|---|---|---|---|
| * SLP-PC92H (iodine value: not more than 45); product of Tsuji Oil Mill Co., Ltd. | | | |

### Test Example 1-3

Compositions for external use were prepared according to the formulations shown in Table 3. Specifically, predetermined amounts of phospholipid, bioactive component, and oligoglycol ether were heated to obtain a solution, which was then mixed with purified water that had been separately heated. Further, a carboxyvinyl polymer that had been previously swollen with water was added to the above-obtained mixture, followed by addition of triethanolamine, to prepare the compositions of Examples 1-6 to 1-14.

When using any of the compositions of Examples 1-6 to 1-14, the percutaneous absorbability of bioactive components contained therein was excellent. In particular, it was confirmed that the percutaneous absorbability of bioactive components was further improved when using the compositions containing at least 30 wt.% of ethoxy diglycol were used.

**Table 3**

| g/100 g | Ex. 16 | Ex. -1-7 | Ex. 1-8 | Ex. 1-9 | Ex. 1-10 | Ex. 1-11 | Ex. 1-12 | Ex. 1-13 | Ex. 1-14 |
|---|---|---|---|---|---|---|---|---|---|
| Hydrogenated soybean phospholipid *1 | 5 | 1 | 10 | 0.1 | - | - | - | - | - |
| Soybean Phospholipid *2 | - | - | - | - | 4 | 1 | 8 | 0.01 | 8 |
| Ethoxy diglycol | 40 | 25 | 50 | 30 | 40 | 25 | 50 | 30 | 30 |
| Retinol palmitate | 0.1 | - | - | - | - | - | - | - | - |
| Arbutin | - | 3 | - | - | - | - | - | - | - |
| Tocopherol acetate | - | - | 0.2 | - | - | - | - | - | - |
| Nicotinic acid amide | - | - | - | 1 | - | - | - | - | - |
| Lidocaine | - | - | - | - | 2 | - | - | - | - |
| Prednisolone valerate acetate | - | - | - | - | - | 1.5 | - | - | - |
| Indomethacin | - | - | - | - | - | - | 3.75 | - | - |
| Felbinac | - | - | - | - | - | - | - | 3 | - |
| Caffeine | - | - | - | - | - | - | - | - | 2 |
| Carboxyvinyl polymer | 0.5 | 0.5 | 0.5 | 0.5 | 0.5 | 0.5 | 0.5 | 0.5 | 0.5 |
| Triethanolamine | q.s. | q.s. | q.s. | q.s. | q.s. | q.s. | q.s. | q.s. | q.s. |
| Purified water | Balance | Balance | Balance | Balance | Balance | Balance | Balance | Balance | Balance |

| | | | | | | | | | |
|---|---|---|---|---|---|---|---|---|---|
| *1: SLP-PC92H (iodine value: not more than 45); product of Tsuji Oil Mill Co., Ltd. *2: BASIS LS-60H (iodine value: not more than 10); product of The Nisshin OilliO Group, Ltd. | | | | | | | | | |

### Test Example 1-4

Compositions for external use were prepared according to the formulations shown in Table 4. Specifically, predetermined amounts of phospholipid, bioactive component, oligoglycol ether, glyceryl tri-2-ethylhexanoate, and polyhydric alcohol were heated to obtain a solution, which was then mixed with purified water that had been separately heated. Further, a carboxyvinyl polymer that had been previously swollen with water was added to the above-obtained mixture, followed by addition of triethanolamine, to prepare the compositions of Examples 1-15 to 1-18.

When using any of the compositions of Examples 1-15 to 1-18, the percutaneous absorbability of bioactive components contained therein was excellent.

**Table 4**

| g/100 g | Ex. 1-15 | Ex. 1-16 | Ex. 1-17 | Ex. 1-18 |
|---|---|---|---|---|
| Hydrogenated soybean phospholipid * | 5 | 1 | 10 | 0.1 |
| Ethoxy diglycol | 22 | 25 | 50 | 30 |
| Retinol palmitate | 0.1 | - | - | - |
| Arbutin | - | 3 | - | - |
| Tocopherol acetate | - | - | 0.2 | - |
| Nicotinamide | - | - | - | 1 |
| Glycerol | 5 | 5 | 5 | 5 |
| Glyceryl tri-2-ethylhexanoate | 5 | 5 | 5 | 5 |
| Carboxyvinyl polymer | 0.5 | 0.5 | 0.5 | 0.5 |
| Triethanolamine | q.s. | q.s. | q.s. | q.s. |
| Purified water | Balance | Balance | Balance | Balance |

| | | | | |
|---|---|---|---|---|
| * Lecinol S-10E (iodine value: not more than 10); Nikko Chemicals Co., Ltd. | | | | |

### Test Example 1-5

Compositions for external use were prepared according to the formulations shown in Table 5. Specifically, predetermined amounts of phospholipid or surfactant, tri(capryl-capric acid)glyceryl solution of d-δ-tocopheryl retinoate, and oligoglycol ether were mixed to obtain a solution, and purified water that had been separately heated was mixed with the resulting solution, to prepare the compositions of Example 1-19 and Comparative Example 1-4, all of which are liposome compositions. Each of the compositions was tested as follows. Ten milliliters of phosphate buffer (pH 7.4) was added to the reservoir compartment of a vertical Franz cell (radius: 0.75 cm), and full-thickness skin from a hairless mouse (HR-1 strain, 7-week-old, male), from which the fat had been removed, was fixed between the cells. Thereafter, 1 ml of the composition was added to the donor compartment. Twenty four hours after adding the composition, the full-thickness skin was removed from the cell. After washing the surface of the full-thickness skin with purified water and ethyl acetate, the full-thickness skin was homogenized to extract, with ethyl acetate, d-δ-tocopheryl retinoate contained in the full-thickness skin. After performing deproteinization, neutralization, gel filtration, centrifugal separation, and/or other treatment, the amount of extracted d-δ-tocopheryl retinoate was measured by HPLC.

Table 5 shows the results. In Table 5, the retained amount (µg/cm²) means the amount (µg) of d-δ-tocopheryl retinoate retained 24 hours after adding the composition, per cm² of the hairless mouse full-thickness skin.

**Table 5**

| g/100 g | Ex.1-19 | Comp.Ex.1-4 |
|---|---|---|
| Hydrogenated soybean phospholipid * | 1 | - |
| d-δ-Tocopheryl retinoate | 0.25 | 0.25 |
| Ethoxy diglycol | 28 | 28 |
| Polyoxyethylene hydrogenated castor oil 60 | - | 0.1 |
| Tri(capryl-capric acid)glyceryl | 2.25 | 2.25 |
| Purified water | Balance | Balance |
| Retained amount (µg/cm²) | 24.3 | 5.0 |

| | | |
|---|---|---|
| * SLP-PC92H (iodine value: not more than 45); product of Tsuji Oil Mill Co., Ltd. | | |

It was revealed that, when using the composition of Example 1-19 containing a phospholipid, the retained amount of d-δ-tocopheryl retinoate was remarkably greater (4.9 times greater) than that obtained when using the composition of Comparative Example 1-4 containing a surfactant. In this test, since d-δ-tocopheryl retinoate is sparingly soluble in phosphate buffer, the amount that permeated through the stratum corneum and was retained in the skin was used as an index of the amount of percutaneous absorption.

### Test Example 1-6

Compositions for external use were prepared according to the formulations shown in Table 6. Specifically, predetermined amounts of phospholipid, oligoglycol ether, and polyhydric alcohol were heated to obtain a solution, and a predetermined amount of aqueous caffeine solution that had been separately heated was added to the above-obtained solution, to prepare the compositions of Examples 1-20 to 1-23 and Comparative Examples 1-5 and 1-6, all of which are liposome compositions. Each of the compositions was tested as follows. Ten milliliters of phosphate buffer (pH 7.4) was added to the reservoir compartment of a vertical Franz cell, and full-thickness skin from a hairless mouse (HR-1 strain, 7-week-old, male), from which the fat had been removed, was fixed between the cells. Thereafter, 1 ml of one of the above compositions was added to the donor compartment. Thirty hours after the addition, the caffeine concentration in the phosphate buffer in the reservoir compartment was determined by HPLC.

Table 6 shows the results. In Table 6, the permeated amount (µg/cm²) means the amount (µg) of caffeine that permeated during the 30 hours that followed the addition of the composition, per cm² of the hairless mouse full-thickness skin.

**Table 6**

| g/100 g | Ex. 1-20 | Ex. 1-21 | Ex. 1-22 | Ex. 1-23 | Comp.Ex. 1-24 | Comp.Ex. 1-6 |
|---|---|---|---|---|---|---|
| Hydrogenated soybean phospholipid *1 | 5 | 1 | - | - | 1 | 1 |
| Soybean phospholipid *2 | - | - | 1 | - | - | - |
| Soybean phospholipid *3 | - | - | - | 1 | - | - |
| Caffeine | 2 | 2 | 2 | 2 | 2 | 2 |
| Ethoxy diglycol | 49 | 49 | 49 | 49 | - | - |
| 1,3-Butylene glycol | - | - | - | - | 49 | - |
| Purified water | Balance | Balance | Balance | Balance | Balance | Balance |
| Permeated amount (µg/cm²) | 3893 | 1599 | 7626 | 6987 | 432 | 122 |

| | | | | | | |
|---|---|---|---|---|---|---|
| *1: SLP-PC92H (iodine value: not more than 45); product of Tsuji Oil Mill Co., Ltd. *2: SLP-PC92; product of Tsuji Oil Mill Co., Ltd. *3: SLP-White; product of Tsuji Oil Mill Co., Ltd. | | | | | | |

It was found that, when using the composition of Example 1-21 containing ethoxy diglycol, the permeated amount was 3.8 times greater than that obtained when using the composition of Comparative Example 1-5 containing 1,3-butanediol. Further, it was confirmed, from the results of testing the composition of Comparative Example 1-6, that hydrogenated soybean phospholipid by itself does not have a very strong percutaneous absorption improving effect.

Further, it was revealed, from the results of testing the compositions of Example 1-22 and 1-23, that when using non-hydrogenated soybean phospholipid as a phospholipid, the permeated amount was increased to as much as 4 times that obtained when using the composition of Example 1-21, demonstrating a particularly excellent percutaneous absorption improving effect.

### Test Example 1-7

Compositions for external use were prepared according to the formulations shown in Table 7. Specifically, predetermined amounts of phospholipid, coenzyme Q10, glyceryl tri-2-ethylhexanoate, and oligoglycol ether or glycerol were mixed to obtain a solution, which was then mixed with purified water that had been separately heated. Necessary amounts of other components were added to the above-obtained mixture, to prepare the compositions of Examples 1-24 to 1-26 and Comparative Examples 1-7, all of which are liposome compositions. Each of the compositions was tested as follows. Ten milliliters of glyceryl tri-2-ethylhexanoate was added to the reservoir compartment of a vertical Franz cell (radius: 0.75 cm), and full-thickness skin from a hairless mouse (HR-1 strain, 7-week-old, male), from which the fat had been removed, was fixed between the cells Thereafter, 2 g of one of the above compositions was added to the donor compartment. Two hours after adding the composition, the full-thickness skin was removed from the cell. After washing the surface of the full-thickness skin with purified water and ethyl acetate, the full-thickness skin was homogenized to extract, with ethyl acetate, coenzyme Q10 contained in the sample. The amount of coenzyme Q10 in the obtained extract was determined by HPLC.

Table 7 shows the results. In Table 7, the retained amount (µg/cm²) means the amount (µg) of coenzyme Q10 retained two hours after adding the composition, per cm² of the hairless mouse full-thickness skin.

**Table 7**

| g/100 g | Ex. 1-24 | Ex. 1-25 | Ex. 1-26 | Comp.Ex. 1-7 |
|---|---|---|---|---|
| Partially hydrogenated soybean lecitin *1 | 1 | - | - | - |
| Hydrogenated soybean lecitin *2 | - | 1 | - | 1 |
| Soybean lecitin *3 | - | - | 1 | - |
| Ethoxy diglycol | 69 | 69 | 69 | - |
| Coenzyme Q10 | 0.03 | 0.03 | 0.03 | 0.03 |
| Glycerol | - | - | - | 69 |
| Glyceryl tri-2-ethylhexanoate | 2.97 | 2.97 | 2.97 | 2.97 |
| Purified water | q.s. | q.s. | q.s. | q.s. |
| Retained amount (µg/cm²) | 1.58 | 0.89 | 2.10 | 0.25 |

| | | | | |
|---|---|---|---|---|
| *1: SLP-PC92H (iodine value: not more than 45); product of Tsuji Oil Mill Co., Ltd. *2: SLP-PC70; product of Tsuji Oil Mill Co., Ltd. *3: Lecinol S-10E(iodine value: not more than 10); product of Nikko Chemicals Co., Ltd. | | | | |

When using the compositions of Example 1-24 to 1-26 containing ethoxy diglycol, the retained amount of coenzyme Q10 was remarkably greater (3.6 to 8.4 times greater) than that obtained when using the composition of Comparative Example 1-7 containing glycerol. It was also revealed that use of lecithin with a lower hydrogenation degree results in higher percutaneous absorbability of coenzyme Q10.

### Test Example 1-8

Compositions for external use were prepared according to the formulations shown in Table 8. Specifically, predetermined amounts of phospholipid, tri(capryl-capric acid)glyceryl solution of d-δ-tocopheryl retinoate, and oligoglycol ether were heated to obtain a solution, which was then mixed with purified water that had been separately heated. Further, a carboxyvinyl polymer that had been previously swollen with water was added to the above-obtained mixture, followed by addition of triethanolamine, to prepare the compositions of Examples 1-27 to 1-36.

When using any of the compositions of Examples 1-27 to 1-36, the percutaneous absorbability of d-δ-tocopheryl retinoate contained therein was excellent. In particular, it was confirmed that the percutaneous absorbability of d-δ-tocopheryl retinoate was further improved when the compositions containing 25 to 80 wt.%, especially 30 to 70 wt.%, of ethoxy diglycol were used.

**Table 8**

| g/100 g | Ex. 1-27 | Ex. 1-28 | Ex. 1-29 | Ex. 1-30 | Ex. 1-31 | Ex. 1-32 | Ex. 1-33 | Ex. 1-34 | Ex. 1-35 | Ex. 1-36 |
|---|---|---|---|---|---|---|---|---|---|---|
| Soybean phospholipid* | 0.1 | 8 | 0.1 | 8 | 0.1 | 8 | 0.1 | 8 | 0.1 | 8 |
| Ethoxy diglycol | 22 | 22 | 25 | 25 | 30 | 30 | 70 | 70 | 80 | 80 |
| d-δ-Tocopheryl retinoate | 0.25 | 0.25 | 0.25 | 0.25 | 0.25 | 0.25 | 0.25 | 0.25 | 0.25 | 0.25 |
| Tri(capryl-capric acid)glyceryl | 2.25 | 2.25 | 2.25 | 2.25 | 2.25 | 2.25 | 2.25 | 2.25 | 2.25 | 2.25 |
| Carboxyvinyl polymer | 0.5 | 0.5 | 0.5 | 0.5 | 0.5 | 0.5 | 0.5 | 0.5 | 0.5 | 0.5 |
| Triethanol-amine | q.s. | q.s. | q.s. | q.s. | q.s. | q.s. | q.s. | q.s. | q.s. | q.s. |
| Purified water | Balance | Balance | Balance | Balance | Balance | Balance | Balance | Balance | Balance | Balance |

| | | | | | | | | | | |
|---|---|---|---|---|---|---|---|---|---|---|
| * SLP-PC70; product of Tsuji Oil Mill Co., Ltd. | | | | | | | | | | |

### Test Example 1-9

Compositions for external use were prepared according to the formulations shown in Table 9. Specifically, predetermined amounts of phospholipid, coenzyme Q10, glyceryl tri-2-ethylhexanoate, and oligoglycol ether were heated to obtain a solution, which was then mixed with purified water that had been separately heated. Further, a carboxyvinyl polymer that had been previously swollen with water was added to the above-obtained mixture, followed by addition of triethanolamine, to prepare the compositions of Examples 1-37 to 1-46.

When using any of the compositions of Examples 1-37 to 1-46, the percutaneous absorbability of coenzyme Q10 contained therein was excellent. In particular, it was confirmed that the percutaneous absorbability of coenzyme Q10 was further improved when compositions containing 25 to 80 wt.%, and especially 30 to 70 wt.%, of ethoxy diglycol were used.

**Table 9**

| g/100 g | Ex. 1-37 | Ex. 1-38 | Ex. 1-39 | Ex. 1-40 | Ex. 1-41 | Ex. 1-42 | Ex. 1-43 | Ex. 1-44 | Ex. 1-45 | Ex. 1-46 |
|---|---|---|---|---|---|---|---|---|---|---|
| Soybean phospholipid * | 0.1 | 8 | 0.1 | 8 | 0.1 | 8 | 0.1 | 8 | 0.1 | 8 |
| Ethoxy diglycol | 22 | 22 | 25 | 25 | 30 | 30 | 70 | 70 | 80 | 80 |
| Coenzyme Q10 | 0.03 | 0.03 | 0.03 | 0.03 | 0.03 | 0.03 | 0.03 | 0.03 | 0.03 | 0.03 |
| Glyceryl tri-2-ethylhexanoate | 2.97 | 2.97 | 2.97 | 2.97 | 2.97 | 2.97 | 2.97 | 2.97 | 2.97 | 2.97 |
| Carboxyvinyl polymer | 0.5 | 0.5 | 0.5 | 0.5 | 0.5 | 0.5 | 0.5 | 0.5 | 0.5 | 0.5 |
| Triethanolamine | q.s. | q.s. | q.s. | q.s. | q.s. | q.s. | q.s. | q.s. | q.s. | q.s. |
| Purified water | Balance | Balance | Balance | Balance | Balance | Balance | Balance | Balance | Balance | Balance |

| | | | | | | | | | | |
|---|---|---|---|---|---|---|---|---|---|---|
| * SLP-PC70; product of Tsuji Oil Mill Co., Ltd. | | | | | | | | | | |

### Test Example 2-1

Compositions for external use were prepared according to the formulations shown in Table 10. Specifically, predetermined amounts of phospholipid, tri(capryl-capric acid)glyceryl solution of d-δ-tocopheryl retinoate, oligoglycol ether, and polyhydric alcohol were mixed to obtain a solution, which was then mixed with purified water that had been separately heated, to prepare the compositions of Example 2-1 and Comparative Example 2-1, both of which are liposome compositions. The compositions were evaluated with respect to the percutaneous absorbability of d-δ-tocopheryl retinoate in the same manner as in Test Example 1-5. Table 10 shows the results.

**Table 10**

| g/100 g | Ex.2-1 | Comp.Ex. 2-1 |
|---|---|---|
| Hydrogenated soybean phospholipid * | 1 | 1 |
| d-δ-Tocopheryl retinoate | 0.25 | 0.25 |
| Ethoxy diglycol | 5 | - |
| 1,3-Butylene glycol | 38.8 | - |
| Dipropylene glycol | - | 43.8 |
| Tri(capryl-capric acid)glyceryl | 2.25 | 2.25 |
| Purified water | Balance | Balance |
| Retained amount (µg/cm²) | 0.33 | 0.11 |

| | | |
|---|---|---|
| * SLP-PC92H(iodine value: not more than 45); product of Tsuji Oil Mill Co., Ltd. | | |

When using the composition of Example 2-1, the retained amount of d-δ-tocopheryl retinoate was remarkably greater (3 times greater) than that obtained when using the composition of Comparative Example 2-1. In this test, since d-δ-tocopheryl retinoate is sparingly soluble in phosphate buffer, the amount of d-δ-tocopheryl retinoate that permeated through the stratum corneum and was retained in the skin was used as an index of the amount of percutaneous absorption.

### Test Example 2-2

Compositions for external use were prepared according to the formulations shown in Table 11. Specifically, predetermined amounts of phospholipid, tri(capryl-capric acid)glyceryl solution of d-δ-tocopheryl retinoate, ascorbyl tetra-2-hexyldecanoate, coenzyme Q10, oligoglycol ether, and polyhydric alcohol were mixed to obtain a solution, which was then mixed with purified water that had been separately heated. An acryloyldimethyl taurine ammonium/VP copolymer that had been previously swollen with water was added to the above-obtained mixture, to prepare the compositions of Example 2-2 to 2-5.

When using any of the compositions of Examples 2-2 to 2-5, the percutaneous absorbability of bioactive components contained therein was excellent.

**Table 11**

| g/100 g | Ex.2-2 | Ex.2-3 | Ex.2-4 | Ex.2-5 |
|---|---|---|---|---|
| Hydrogenated soybean phospholipid * | 1 | 5 | 1 | 1 |
| Ethoxy diglycol | 5 | 6 | 5 | 6 |
| 1,3-Butylene glycol | 40 | - | 40 | - |
| Dipropylene glycol | - | 39 | - | 39 |
| d-δ-Tocopheryl retinoate | 0.25 | 0.5 | - | - |
| Ascorbyl tetra-2-hexyldecanoate | 1 | 1 | - | - |
| Coenzyme Q10 | - | - | 0.03 | 0.03 |
| Tri(capryl-capric acid)glyceryl | 2.25 | 4.5 | - | - |
| Acryloyldimethyl taurine ammonium/VP copolymer | 1 | 1 | 1 | 1 |
| Purified water | Balance | Balance | Balance | Balance |

| | | | | |
|---|---|---|---|---|
| * SLP-PC92H (iodine value: not more than 45); product of Tsuji Oil Mill Co., Ltd. | | | | |

### Test Example 2-3

Compositions for external use were prepared according to the formulations shown in Tables 12 and 13. Specifically, predetermined amounts of phospholipid, bioactive component, oligoglycol ether, glyceryl tri-2-ethylhexanoate, and polyhydric alcohol were mixed to obtain a solution, which was then mixed with purified water that had been separately heated. Further, a carboxyvinyl polymer that had been previously swollen with water was added to the above-obtained mixture, followed by addition of triethanolamine, to prepare the compositions of Examples 2-6 to 2-18.

The results of testing the obtained compositions revealed that, when using any of the compositions of Example 2-6 to 2-18, which contained 0.01 to 8 wt.% of phospholipid and 0.01 to 6.5 wt.% of ethoxy diglycol, the percutaneous absorbability of bioactive components contained therein was excellent.

**Table 12**

| g/100 g | Ex. 2-6 | Ex. 2-7 | Ex. 2-8 | Ex. 2-9 | Ex. 2-10 | Ex. 2-11 | Ex. 2-12 | Ex. 2-13 | Ex. 2-14 |
|---|---|---|---|---|---|---|---|---|---|
| Hydrogenated soybean phospholipid *1 | 4 | 1 | 8 | 0.01 | - | - | - | - | - |
| Soybean phospholipid *2 | | - | - | - | 4 | 1 | 8 | 0.01 | 8 |
| Ethoxy diglycol | 3 | 1 | 6.5 | 0.1 | 6.5 | 3 | 1 | 0.1 | 6 |
| Retinol palmitate | 0,1- | - | - | - | - | - | - | - | - |
| Arbutin | - | 3 | - | - | - | - | - | - | - |
| Tocopherol acetate | - | - | 0.2 | - | - | - | - | - | - |
| Nicotinic acid amide | - | - | - | 1 | - | - | - | - | - |
| Lidocaine | - | - | - | - | 2 | - | - | - | - |
| Prednisolone valerate acetate | - | - | - | - | - | 1.5 | - | - | - |
| Indomethacin | - | - | - | - | - | - | 3.75 | - | - |
| Felbinac | - | - | - | - | - | - | - | 3 | - |
| Caffeine | - | - | - | - | - | - | - | - | 2 |
| 1,3-Butylene glycol | 10 | 10 | 10 | 10 | 10 | 10 | 10 | 10 | 10 |
| Carboxyvinyl polymer | 0.5 | 0.5 | 0.5 | 0.5 | 0.5 | 0.5 | 0.5 | 0.5 | 0.5 |
| Triethanolamine | q.s. | q.s. | q.s. | q.s. | q.s. | q.s. | q.s. | q.s. | q.s. |
| Purified water | Balance | Balance | Balance | Balance | Balance | Balance | Balance | Balance | Balance |

| | | | | | | | | | |
|---|---|---|---|---|---|---|---|---|---|
| *1: SLP-PC92H (iodine value: not more than 45); product of Tsuji Oil Mill Co., Ltd. *2: BASIS LS-60H (iodine value: not more than 10); product of The Nisshin OilliO Group, Ltd. | | | | | | | | | |

**Table 13**

| | Ex. 2-15 | Ex. 2-16 | Ex. 2-17 | Ex. 2-18 |
|---|---|---|---|---|
| Hydrogenated soybean phospholipid * | 4 | 1 | 8 | 0.01 |
| Ethoxy diglycol | 6.5 | 3 | 1 | 0.1 |
| Retinol palmitate | 0.1 | - | - | - |
| Arbutin | - | 7 | - | - |
| Tocopherol acetate | - | - | 0.5 | - |
| nicotinamide | - | - | - | 1 |
| 1,3-Butylene glycol | 10 | 10 | 10 | 10 |
| Glyceryl tri-2-ethylhexanoate | 10 | 10 | 10 | 10 |
| Carboxyvinyl polymer | 0.5 | 0.5 | 0.5 | 0.5 |
| Triethanolamine | q.s. | q.s. | q.s. | q.s. |
| Purified water | Balance | Balance | Balance | Balance |

| | | | | |
|---|---|---|---|---|
| * Lecinol S-10E (iodine value: not more than 10); product of Nikko Chemicals Co., Ltd. | | | | |

### Test Example 2-4

Compositions for external use were prepared according to the formulations shown in Table 14. Specifically, predetermined amounts of phospholipid, oligoglycol ether, and polyhydric alcohol were heated to obtain a solution, which was then mixed with an aqueous solution of caffeine that had been separately heated, to prepare the compositions (liposome compositions) of Example 2-19 and Comparative Examples 2-2 and 2-3. The compositions were evaluated with respect to the percutaneous absorbability of caffeine in the same manner as in Test Example 1-6. Table 14 shows the results.

**Table 14**

| g/100 g | Ex. 2-19 | Comp.Ex. 2-2 | Comp.Ex. 2-3 |
|---|---|---|---|
| Hydrogenated soybean phospholipid * | 1 | 1 | 1 |
| Caffeine | 2 | 2 | 2 |
| Ethoxy diglycol | 5 | - | - |
| 1,3-Butylene glycol | 44 | 49 | - |
| Purified water | Balance | Balance | Balance |
| Permeated amount (µg/cm²) | 652 | 423 | 122 |

| | | | |
|---|---|---|---|
| * SLP-PC92H (iodine value: not more than 45); product of Tsuji Oil Mill Co., Ltd. | | | |

It was revealed that, when using the composition of Example 2-19 containing ethoxy diglycol, the permeated amount was as much as 1.5 times or more that obtained when using the composition of Comparative Example 2-2 containing 1,3-butanediol.

It was confirmed, from the results of testing the composition of Comparative Example 2-3, that hydrogenated soybean phospholipid by itself does not have a very strong percutaneous absorption improving effect.

### Test Example 2-5

Compositions for external use were prepared according to the formulations shown in Tables 15 and 16. Specifically, predetermined amounts of phospholipid, tri(capryl-capric acid)glyceryl solution of d-δ-tocopheryl retinoate, oligoglycol ether, and polyhydric alcohol were heated to obtain a solution, which was then mixed with purified water that had been separately heated. Further, a carboxyvinyl polymer that had been previously swollen with water was added to the above-obtained mixture, followed by addition of triethanolamine, to prepare the compositions of Examples 2-20 to 2-33.

When using any of the compositions of Examples 2-20 to 2-33, the percutaneous absorbability of d-δ-tocopheryl retinoate contained therein was excellent. It was also confirmed that the percutaneous absorbability of d-δ-tocopheryl retinoate was particularly excellent when the compositions containing 0.1 to 7.5 wt.% of phospholipid and 0.1 to 6 wt.% of ethoxy diglycol were used, and that the percutaneous absorbability of d-δ-tocopheryl retinoate was even more excellent when the compositions containing 1 to 7 wt.% of phospholipid and 1 to 5 wt.% of ethoxy diglycol were used.

**Table 15**

| g/100 g | Ex. 2-20 | Ex. 2-21 | Ex. 2-22 | Ex. 2-23 | Ex. 2-24 | Ex. 2-25 | Ex. 2-26 |
|---|---|---|---|---|---|---|---|
| Soybean phospholipid * | 4 | 4 | 4 | 4 | 4 | 4 | 4 |
| Ethoxy diglycol | 0.01 | 0.1 | 1 | 3 | 5 | 6 | 6.5 |
| d-δ-Tocopheryl retinoate | 0.25 | 0.25 | 0.25 | 0.25 | 0.25 | 0.25 | 0.25 |
| Glycerol | 3 | 3 | 3 | 3 | 3 | 3 | 3 |
| Tri(capryl-capric acid)glyceryl | 2.25 | 2.25 | 2.25 | 2.25 | 2.25 | 2.25 | 2.25 |
| Carboxyvinyl polymer | 0.5 | 0.5 | 0.5 | 0.5 | 0.5 | 0.5 | 0.5 |
| Triethanolamine | q.s. | q.s. | q.s. | q.s. | q.s. | q.s. | q.s. |
| Purified water | Balance | Balance | Balance | Balance | Balance | Balance | Balance |

| | | | | | | | |
|---|---|---|---|---|---|---|---|
| * SLP-PC70; product of Tsuji Oil Mill Co., Ltd. | | | | | | | |

**Table 16**

| g/100 g | Ex. 2-27 | Ex. 2-28 | Ex. 2-29 | Ex. 2 -30 | Ex. 2-31 | Ex. 2-32 | Ex. 2-33 |
|---|---|---|---|---|---|---|---|
| Soybean phospholipid * | 0.01 | 0.1 | 1 | 4 | 7 | 7.5 | 8 |
| Ethoxy diglycol | 5 | 5 | 5 | 5 | 5 | 5 | 5 |
| d-δ-Tocopheryl retinoate | 0.25 | 0.25 | 0.25 | 0.25 | 0.25 | 0.25 | 0.25 |
| Glycerol | 3 | 3 | 3 | 3 | 3 | 3 | 3 |
| Tri(capryl-capric acid)glyceryl | 2.25 | 2.25 | 2.25 | 2.25 | 2.25 | 2.25 | 2.25 |
| Carboxyvinyl polymer | 0.5 | 0.5 | 0.5 | 0.5 | 0.5 | 0.5 | 0.5 |
| Triethanolamine | q.s. | q.s. | q.s. | q.s. | q.s. | q.s. | q.s. |
| Purified water | Balance | Balance | Balance | Balance | Balance | Balance | Balance |

| | | | | | | | |
|---|---|---|---|---|---|---|---|
| * SLP-PC70; product of Tsuji Oil Mill Co., Ltd. | | | | | | | |

### Test Example 2-6

Compositions for external use were prepared according to the formulations shown in Tables 17 and 18. Specifically, predetermined amounts of phospholipid, coenzyme Q10, glyceryl tri-2-ethylhexanoate, and oligoglycol ether were heated to obtain a solution, which was then mixed with purified water that had been separately heated. Further, a carboxyvinyl polymer that had been previously swollen with water was added to the above-obtained mixture, followed by addition of triethanolamine, to prepare the compositions of Examples 2-34 to 2-47.

When using any of the compositions of Examples 2-34 to 2-47, the percutaneous absorbability of coenzyme Q10 contained therein was excellent. It was also confirmed that the percutaneous absorbability of coenzyme Q10 was particularly excellent when the compositions containing 0.1 to 7.5 wt.% of phospholipid and 0.1 to 6 wt.% of ethoxy diglycol were used, and that the percutaneous absorbability of coenzyme Q10 was even more excellent when the compositions containing 1 to 7 wt.% of phospholipid and 1 to 5 wt.% of ethoxy diglycol were used.

**Table 17**

| g/100 g | Ex. 2-34 | Ex. 2-35 | Ex. 2-36 | Ex. 2-37 | Ex. 2-38 | Ex. 2-39 | Ex. 2-40 |
|---|---|---|---|---|---|---|---|
| Soybean phospholipid * | 4 | 4 | 4 | 4 | 4 | 4 | 4 |
| Ethoxy diglycol | 0.01 | 0.1 | 1 | 3 | 5 | 6 | 6.5 |
| Coenzyme Q10 | 0.03 | 0.03 | 0.03 | 0.03 | 0.03 | 0.03 | 0.03 |
| Glycerol | 3 | 3 | 3 | 3 | 3 | 3 | 3 |
| Glyceryl tri-2-ethylhexanoate | 2.97 | 2.97 | 2.97 | 2.97 | 2.97 | 2.97 | 2.97 |
| Carboxyvinyl polymer | 0.5 | 0.5 | 0.5 | 0.5 | 0.5 | 0.5 | 0.5 |
| Triethanolamine | q.s. | q.s. | q.s. | q.s. | q.s. | q.s. | q.s. |
| Purified water | Balance | Balance | Balance | Balance | Balance | Balance | Balance |

| | | | | | | | |
|---|---|---|---|---|---|---|---|
| * SLP-PC70; product of Tsuji Oil Mill Co., Ltd. | | | | | | | |

**Table 18**

| g/100 g | Ex. 2-41 | Ex. 2-92 | Ex. 2-43 | Ex. 2-44 | Ex. 2-45 | Ex. 2-46 | Ex. 2-97 |
|---|---|---|---|---|---|---|---|
| Soybean phospholipid * | 0.01 | 0.1 | 1 | 4 | 7 | 7.5 | 8 |
| Ethoxy diglycol | 5 | 5 | 5 | 5 | 5 | 5 | 5 |
| Coenzyme Q10 | 0.03 | 0.03 | 0.03 | 0.03 | 0.03 | 0.03 | 0.03 |
| Glycerol | 3 | 3 | 3 | 3 | 3 | 3 | 3 |
| Glyceryl tri-2-ethylhexanoate | 2.97 | 2.97 | 2.97 | 2.97 | 2.97 | 2.97 | 2.97 |
| Carboxyvinyl polymer | 0.5 | 0.5 | 0.5 | 0.5 | 0.5 | 0.5 | 0.5 |
| Triethanolamine | q.s. | q.s. | q.s. | q.s. | q.s. | q.s. | q.s. |
| Purified water | Balance | Balance | Balance | Balance | Balance | Balance | Balance |

| | | | | | | | |
|---|---|---|---|---|---|---|---|
| * SLP-PC70; product of Tsuji Oil Mill Co., Ltd. | | | | | | | |

### Test Example 3-1

Compositions for external use were prepared according to the formulations shown in Table 19. Specifically, predetermined amounts of phospholipid and oligoglycol ether were heated to obtain a solution, which was then mixed with purified water that had been separately heated. Further, an acryloyldimethyltaurine ammonium-VP copolymer, which had been previously swollen with water, and sodium hyaluronate were added to the above-obtained mixture, to prepare the compositions of Example 3-1, Comparative Example 3-1, and Control Example 3, among which the compositions of Example 3-1 and Comparative Example 3-1 are liposome compositions. Each of the compositions was tested as follows. Ten milliliters of phosphate buffer (pH 7.4) was added to the reservoir compartment of a vertical Franz cell (radius: 0.75 cm), and full-thickness skin from a hairless mouse (HR-1 strain, 7-week-old, male), from which the fat had been removed, was fixed between the cells. Thereafter, 1 ml of one of the above compositions was added to the donor compartment. Twenty four hours after adding the composition, the full-thickness skin was removed from the cell. After washing the surface of the full-thickness skin with purified water and acetone, the full-thickness skin was homogenized to extract, with purified water, hyaluronic acid contained in the full-thickness skin. The amount of extracted hyaluronic acid was determined using a hyaluronic acid measurement kit (Seikagaku Corp.). Further, 1 g of purified water was added to the donor compartment of a Franz cell, and a test was performed under the same conditions as above (Control Example 3), to measure the amount of hyaluronic acid that is naturally present in the full-thickness skin. The amount (Permeated amount: µg/cm²) of hyaluronic acid that permeated through the full-thickness skin was calculated from the measurements obtained in Control Example 3.

Table 19 shows the results.

**Table 19**

| g/100 ml | Ex. 3-1 | Comp.Ex. 3-1 | Cont.Ex. 3 |
|---|---|---|---|
| Partially hydrogenated soybean lecitin (SLP-PC92H) | 1 | - | - |
| Ethoxy diglycol | 49 | 49 | - |
| Sodium hyaluronate | 0.1 | 0.1 | - |
| Acryloyldimethyl taurine ammonium/VP copolymer | 0.3 | 0.3 | - |
| Purified water | Balance | Balance | 100 |
| Retained amount (µg/cm²) | 13.6 | 7.7 | 3.4 |
| Permeated amount(µg/cm²) | 10.2 | 4.3 | - |

The above results also demonstrate that, although it has been said that hyaluronic acid does not permeate into the skin, the composition of Example 3-1 enables hyaluronic acid to permeate into the skin.

### Test Example 3-2

Compositions for external use were prepared according to the formulation shown in Table 20. Specifically, predetermined amounts of phospholipid, oligoglycol ether, and polyhydric alcohol were heated to obtain a solution, which was then mixed with an aqueous solution of ascorbic acid that had been separately heated. Predetermined amounts of other necessary components were further added to the above-obtained mixture, to prepare the compositions of Examples 3-2 to 3-4 and Comparative Examples 3-2 to 3-4, which are liposome compositions except for the composition of Comparative Example 3-4. The compositions were evaluated with respect to the percutaneous absorbability of ascorbic acid, in the same manner as in Test Example 1-1. Table 20 shows the results.

**Table 20**

| g/100 g | Ex. 3-2 | Ex. 3-3 | Comp.Ex. 3-2 | Comp.Ex. 3-3 | Comp.Ex. 3-4 |
|---|---|---|---|---|---|
| Hydrogenated soybean phospholipid *1 | 5 | - | 5 | 5 | - |
| Soybean phospholipid *2 | - | 1 | 5 | 5 | - |
| Ascorbic acid | 10 | 20 | 10 | 10 | 20 |
| Ethoxy diglycol | 43 | 49 | - | - | 53 |
| Glycerol | - | - | 43 | - | - |
| 1,3-Butylene glycol | - | - | - | 43 | - |
| Oleylamine | 1 | 1 | - | - | - |
| Diglycerol | - | - | - | - | 4 |
| Trimethylglycine | - | - | - | - | 3 |
| Purified water | Balance | Balance | Balance | Balance | Balance |
| Permeated amount (30 hours) µg/cm² | 1118 | 6350 | 359 | 397 | 174 |

| | | | | | |
|---|---|---|---|---|---|
| *1 SLP-PC92H (iodine value: not more than 45); product of Tsuji Oil Mill Co., Ltd. *2 SLP-PC70; product of Tsuji Oil Mill Co., Ltd. | | | | | |

When using the composition of Example 3-2, the permeated amount was greater (2.8 times or more greater) than that obtained when using the composition of Comparative Example 3-2 or 3-3, and was remarkably greater (6.4 times or more greater) than that obtained when using the composition of Comparative Example 3-4. It was also confirmed, from the results of testing the composition of Example 3-3, that use of non-hydrogenated soybean phospholipid further improves the percutaneous absorbability of ascorbic acid.

### Test Example 3-3

Compositions for external use were prepared according to the formulations shown in Table 21. Specifically, predetermined amounts of phospholipid, tri(capryl-capric acid)glyceryl solution of d-δ-tocopheryl retinoate, oligoglycol ether, and polyhydric alcohol were mixed to obtain a solution, which was then mixed with purified water that had been separately heated, to prepare the compositions of Example 3-4 and Comparative Example 3-5, which are liposome compositions. The compositions were evaluated with respect to the percutaneous absorbability of d-δ-tocopheryl retinoate in the same manner as in Test Example 1-5. Table 21 shows the results.

**Table 21**

| g/100 g | Ex. 3-4 | Comp. Ex. 3-5 |
|---|---|---|
| Hydrogenated soybean phospholipid* | 1 | 1 |
| d-δ-Tocopheryl retinoate | 0.25 | 0.25 |
| Ethoxy diglycol | 5 | - |
| 1,3-Butylene glycol | 38.8 | - |
| Dipropylene glycol | - | 43.8 |
| Tri(capryl-capric acid)glyceryl | 2.25 | 2.25 |
| Purified water | Balance | Balance |
| Retained amount (µg/cm²) | 0.33 | 0.11 |

| | | |
|---|---|---|
| * SLP-PC92H (iodine value: not more than 45); product of Tsuji Oil Mill Co., Ltd. | | |

It was revealed that, when using the composition of Example 3-4 containing ethoxy diglycol, the retained amount was 3 times that obtained when using the composition of Comparative Example 3-5 containing dipropylene glycol. This result demonstrates that the composition of Example 3-4 has remarkably improved percutaneous absorbability.

### Test Example 3-4

Compositions for external use were prepared according to the formulations shown in Table 22. Specifically, predetermined amounts of phospholipid, tri(capryl-capric acid)glyceryl solution of d-δ-tocopheryl retinoate, glycol ether, and polyhydric alcohol were mixed to obtain a solution, which was then mixed with purified water that had been separately heated, to obtain the compositions of Example 3-5 and Comparative Example 3-6, which are liposome compositions. The compositions were evaluated with respect to the percutaneous absorbability of d-δ-tocopheryl retinoate in the same manner as in Test Example 1-5. Table 22 shows the results. In Table 22, the retained amount (µg/cm²) means the amount (µg) of d-δ-tocopheryl retinoate retained 24 hours after adding the composition, per cm² of the hairless mouse full-thickness skin.

**Table 22**

| g/100 g | Ex. 3-5 | Comp. Ex. 3-6 |
|---|---|---|
| Hydrogenated soybean phospholipid * | 1 | - |
| d-δ-Tocopheryl retinoate | 0.25 | 0.25 |
| Ethoxy diglycol | 28 | 28 |
| Polyoxyethylene hydrogenated castor oil 60 | - | 0.1 |
| Tri(capryl-capric acid)glyceryl | 2.25 | 2.25 |
| Purified water | Balance | Balance |
| Retained amount (µg/cm²) | 24.3 | 5.0 |

| | | |
|---|---|---|
| * SLP-PC92H (iodine value: not more than 45); product of Tsuji Oil Mill Co., Ltd. | | |

When using the composition of Example 3-5 containing a phospholipid, the retained amount was remarkably greater (4.9 times greater) than that obtained when using the composition of Comparative Example 3-6.

### Test Example 3-5

Compositions for external use were prepared according to the formulations shown in Table 23. Specifically, predetermined amounts of phospholipid, oligoglycol ether, and polyhydric alcohol were heated to obtain a solution, and an aqueous solution of caffeine that had been separately heated was mixed with the resulting solution, to prepare the compositions of Examples 3-6 to 3-10 and Comparative Examples 3-7 to 3-8, all of which are liposome compositions. The compositions were evaluated with respect to the percutaneous absorbability of caffeine in the same manner as in Test Example 1-6. Table 23 shows the results.

**Table 23**

| g/100 g | Ex. 3-6 | Ex. 3-7 | Ex. 3-8 | Ex. 3-9 | Ex. 3-10 | Comp. Ex. 3-7 | Comp. Ex. 3-8 |
|---|---|---|---|---|---|---|---|
| Hydrogenated soybean phospholipid *1 | 5 | 1 | - | - | 1 | 1 | 1 |
| Soybean phospholipid *2 | - | - | 1 | - | - | - | - |
| Soybean phospholipid *3 | - | - | - | 1 | 1 | - | - |
| Caffeine | 2 | 2 | 2 | 2 | 2 | 2 | 2 |
| Ethoxy diglycol | 49 | 49 | 49 | 49 | 5 | - | - |
| 1,3-Butylene glycol | - | - | - | - | 44 | 49 | - |
| Purified water | Balance | Balance | Balance | Balance | Balance | Balance | Balance |
| Permeated amount (µg/cm²) | 3893 | 1599 | 7626 | 6987 | 652 | 432 | 122 |

| | | | | | | | |
|---|---|---|---|---|---|---|---|
| *1: SLP-PC92H (iodine value: not more than 45); product of Tsuji Oil Mill Co., Ltd. *2: SLP-PC92; product of Tsuji Oil Mill Co., Ltd. *3: SLP-White; product of Tsuji Oil Mill Co., Ltd. | | | | | | | |

When using the composition of Example 3-7 containing ethoxy diglycol, the permeated amount was remarkably greater (3.8 times greater) than that obtained when using the composition of Comparative Example 3-7 containing 1,3-butanediol. It was confirmed, from the results of testing the composition of Comparative Example 3-8, that hydrogenated soybean phospholipid by itself does not have a very strong percutaneous absorption improving effect.

Further, the results of testing the compositions of Examples 3-8 and 3-9 demonstrate that use of non-hydrogenated soybean phospholipid markedly increases the permeated amount.

### Test Example 3-6

Compositions for external use were prepared according to the formulations shown in Tables 24 to 27 (Examples 3-11 to 3-32). When using any of the compositions of Examples 3-11 to 3-32, the percutaneous absorbability of bioactive components contained therein was excellent.

**Table 24**

| g/100 g | Ex.3-11 | Ex.3-12 | Ex.3-13 |
|---|---|---|---|
| Hydrogenated soybean phospholipid * | 5 | 1 | 10 |
| Ascorbic acid | 10 | 10 | 5 |
| Ethoxy diglycol | 43 | 43 | 25 |
| Oleylamine | 1 | 0.5 | 1 |
| 1,3-Butylene glycol | - | - | 18 |
| Purified water | Balance | Balance | Balance |

| | | | |
|---|---|---|---|
| * SLP-PC92H (iodine value: not more than 45); product of Tsuji Oil Mill Co., Ltd. | | | |

**Table 25**

| g/100 g | Ex. 3-14 | Ex. 3-15 | Ex. 3-16 | Ex. 3-17 | Ex. 3-18 | Ex. 3-19 |
|---|---|---|---|---|---|---|
| Hydrogenated soybean phospholipid *1 | 4 | 5 | 5 | - | 1 | 5 |
| Hydrogenated soybean phospholipid *2 | - | - | - | 4 | 0.1 | - |
| Ethoxy diglycol | 3 | 40 | 22 | 6.5 | 5 | 6 |
| Retinol palmitate | 0.1 | 0.1 | 0.1 | 0.1 | - | - |
| d-δ-Tocopheryl retinoate | - | - | - | - | 0.25 | 0.5 |
| Ascorbyl tetra-2-hexyldecanoate | - | - | - | - | 1 | 1 |
| Glyceryl tri-2-ethylhexanoate | - | - | 5 | 10 | - | - |
| Tri(capryl-capric acid)glyceryl | - | - | - | - | 2.25 | 4.5 |
| Dibutylene glycol | - | - | - | - | - | 39 |
| 1,3-Butylene glycol | 10 | - | - | 10 | 40 | - |
| Glycerol | - | - | 5 | - | - | - |
| Acryloyldimethyl taurine ammonium/VP copolymer | - | - | - | - | 1 | 1 |
| Carboxyvinyl polymer | 0.5 | 0.5 | 0.5 | 0.5 | - | - |
| Triethanolamine | q.s. | q.s. | q.s. | q.s. | - | - |
| Purified water | Balance | Balance | Balance | Balance | Balance | Balance |

| | | | | | | |
|---|---|---|---|---|---|---|
| *1 SLP-PC92H (iodine value: not more than 45); product of Tsuji Oil Mill Co., Ltd. *2 Lecinol S-10E (iodine value: not more than 10); product of Nikko Chemicals Co., Ltd. | | | | | | |

**Table 26**

| g/100 g | Ex. 3-20 | Ex. 3-21 | Ex. 3-22 | Ex. 3-23 | Ex. 3-24 |
|---|---|---|---|---|---|
| Soybean phospholipid *1 | 8 | 8 | 4 | - | - |
| Hydrogenated soybean phospholipid *2 | - | - | - | 10 | - |
| Hydrogenated soybean phospholipid *3 | - | - | - | - | 0.5 |
| Ethoxy diglycol | 30 | 6 | 18 | 68 | 10 |
| Caffeine | 2 | 2 | 1 | - | - |
| Theophylline | - | - | - | 1 | - |
| Theobromine | - | - | - | - | 1 |
| 1,3-Butylene glycol | - | 10 | - | - | - |
| Carboxyvinyl polymer | 0.5 | 0.5 | 0.5 | 0.5 | 0.5 |
| Triethanolamine | q.s. | q.s. | q.s. | q.s. | q.s. |
| Purified water | Balance | Balance | Balance | Balance | Balance |

| | | | | | |
|---|---|---|---|---|---|
| *1 Basis LS-60H (iodine value: not more than 10); product of The Nisshin OilliO Group, Ltd. *2 Lecinol S-10E(iodine value: not more than 10); product of Nikko Chemicals Co., Ltd. *3 SLP-PC92H (iodine value: not more than 45); product of Tsuji Oil Mill Co., Ltd. | | | | | |

**Table 27**

| g/100 g | Ex. 3-25 | Ex. 3-26 | Ex. 3-27 | Ex. 3-28 | Ex. 3-29 | Ex. 3-30 | Ex. 3-31 | Ex. 3-32 |
|---|---|---|---|---|---|---|---|---|
| Hydrogenated soybean phospholipid *1 | 5 | - | 2.5 | 3 | - | 1 | - | - |
| Hydrogenated soybean phospholipid *2 | - | - | - | - | 0.1 | - | - | 5 |
| Soybean phospholipid *3 | - | 1 | - | - | - | - | 2 | - |
| Ethoxy diglycol | 15 | 65 | 20 | 18 | 9 | 60 | 13 | 70 |
| Retinol palmitate | 0.1 | - | - | - | - | - | - | - |
| Retinoic acid | - | 0.005 | - | - | - | - | - | - |
| Retinol | - | - | 0.2 | - | - | - | - | - |
| d-δ-Tocopheryl retinoate | - | - | - | 0.1 | - | - | - | - |
| Retinol acetate | - | - | - | - | 0.05 | - | - | - |
| Ascorbic acid | - | - | - | - | - | 20 | - | - |
| Ascorbic acid glucoside | - | - | - | - | - | - | 3 | - |
| Ascorbyl tetra-2-hexyldecanoate | - | - | - | - | - | - | - | 10 |
| Carboxyvinyl polymer | 0.5 | 0.5 | 0.5 | 0.5 | 0.5 | 0.5 | 0.5 | 0.5 |
| Triethanol-amine | q.s. | q.s. | q.s. | q.s. | q.s. | q.s. | q.s. | q.s. |
| Purified water | Balance | Balance | Balance | Balance | Balance | Balance | Balance | Balance |

| | | | | | | | | |
|---|---|---|---|---|---|---|---|---|
| *1 SLP-PC92H (iodine value: not more than 45); product of Tsuji Oil Mill Co., Ltd. *2 Lecinol S-10E (iodine value: not more than 10); product of Nikko Chemicals Co., Ltd. *3 Basis LS-60H (iodine value: not more than 10); product of The Nisshin OilliO Group, Ltd. | | | | | | | | |

### Test Example 3-7

Compositions for external use were prepared according to the formulations shown in Table 28. Specifically, predetermined amounts of phospholipid, coenzyme Q10, oligoglycol ether, glyceryl tri-2-ethylhexanoate, and polyhydric alcohol were mixed to obtain a solution, which was then mixed with purified water that had been separately heated. Further, predetermined amounts other components were added to the resulting mixture, to obtain the compositions of Examples 3-33 to 3-35 and Comparative Example 3-9, which are liposome compositions. The compositions were evaluated with respect to the percutaneous absorbability of coenzyme Q10 in the same manner as in Test Example 1-7. Table 28 shows the results.

**Table 28**

| g/100 g | Ex. 3-33 | Ex. 3-34 | Ex. 3-35 | Comp.Ex. 3-9 |
|---|---|---|---|---|
| Partially hydrogenated soybean lecitin *1 | 1 | - | - | - |
| Hydrogenated soybean lecitin *2 | - | 1 | - | 1 |
| Soybean lecitin *3 | - | - | 1 | - |
| Ethoxy diglycol | 69 | 69 | 69 | - |
| Coenzyme Q10 | 0.03 | 0.03 | 0.03 | 0.03 |
| Glycerol | - | - | - | 69 |
| Glyceryl tri-2-ethylhexanoate | 2.97 | 2.97 | 2.97 | 2.97 |
| Purified water | q.s. | q.s. | q.s. | q.s. |
| Retained amount (µg) | 2.80 | 1.58 | 3.71 | 0.44 |

| | | | | |
|---|---|---|---|---|
| *1 SLP-PC92H (iodine value: not more than 45); product of Tsuji Oil Mill Co., Ltd. *2 Lecinol S-10E(iodine value: not more than 10); product of Nikko Chemicals Co., Ltd. *3 SLP-PC70; product of Tsuji Oil Mill Co., Ltd. | | | | |

When using the compositions of Examples 3-33 to 3-35 containing ethoxy diglycol, the retained amount of coenzyme Q10 in the skin was remarkably greater (3.6 to 8.4 times greater) than that obtained when using the composition of Comparative Example 3-9 containing glycerol. It was also revealed that use of lecithin with a lower hydrogenation degree resulted in higher percutaneous absorbability of coenzyme Q10. Ubiquinones containing 6 to 9 isoprene units (coenzymes Q6 to Q9) have very similar properties to those of coenzyme Q10, which has 10 isoprene units, and therefore are expected to exhibit substantially the same percutaneous absorption behavior as coenzyme Q10.

### Test Example 3-8

Compositions for external use were prepared according to the formulations shown in Tables 29 and 30 (Examples 3-36 to 3-49). When using any of the compositions of Examples 3-36 to 3-49, the percutaneous absorbability of bioactive components contained therein was excellent.

**Table 29**

| g/100 g | Ex.3-36 | Ex.3-37 | Ex.3-38 |
|---|---|---|---|
| Hydrogenated soybean phospholipid *1 | 1 | 1 | 1 |
| Hydrogenated soybean phospholipid *2 | - | - | 1.5 |
| Ethoxy diglycol | 5 | 6 | 5 |
| Coenzyme Q10 | 0.03 | 0.03 | 0.03 |
| Vitamin A oil | - | - | 0.15 |
| 1,3-Butylene glycol | 40 | - | 10 |
| Dipropylene glycol | - | 39 | 10 |
| Glyceryl tri-2-ethylhexanoate | - | - | 10 |
| Acryloyldimethyl taurine ammonium/VP copolymer | 1 | 1 | - |
| Acrylic acid-alkyl methacrylate copolymer | - | - | 1 |
| Triethanolamine | - | - | 0.1 |
| Purified water | Balance | Balance | Balance |

| | | | |
|---|---|---|---|
| *1 SLP-PC92H (iodine value: not more than 45); product of Tsuji Oil Mill Co., Ltd. *2 Lecinol S-10E (iodine value: not more than 10); product of Nikko Chemicals Co., Ltd. | | | |

**Table 30**

| g/100 g | Ex. 3-39 | Ex. 3-40 | Ex. 3-41 | Ex. 3-42 | Ex. 3-43 | Ex. 3-44 | Ex. 3-45 | Ex. 3-46 | Ex. 3-47 | Ex. 3-48 | Ex. 3-49 |
|---|---|---|---|---|---|---|---|---|---|---|---|
| Hydrogenated soybean phospholipid *1 | - | - | 2.5 | | 0.1 | - | - | - | - | 10 | - |
| Hydrogenated soybean phospholipid *2 | 5 | - | - | 3 | - | 1 | - | - | 4 | - | 0.5 |
| Soybean phospholipid *3 | - | 1 | - | - | - | - | 2 | 0.5 | - | - | - |
| Coenzyme Q6 | 0.03 | 0.3 | 3 | - | - | - | - | - | - | - | - |
| Coenzyme Q7 | - | 0.03 | 0.3 | 3 | - | - | - | - | - | - | - |
| Coenzyme Q8 | - | - | - | 0.03 | 0.3 | 3 | - | - | - | - | - |
| Coenzyme Q9 | - | - | - | - | 0.03 | 0.3 | 3 | - | - | - | - |
| Coenzyme Q10 | - | - | - | - | - | - | - | 0.01 | 0.3 | 1 | 3 |
| Glyceryl tri-2-ethylhexanoate. | 3 | 3 | 10 | 10 | 3 | 10 | 10 | 3 | 3 | 3 | 10 |
| Ethoxy diglycol | 15 | 65 | 20 | 18 | 9 | 60 | 13 | 70 | 18 | 68 | 10 |
| Carboxyvinyl polymer | 0.5 | 0.5 | 0.5 | 0.5 | 0.5 | 0.5 | 0.5 | 0.5 | 0.5 | 0.5 | 0.5 |
| Triethanolamine | q.s. | q.s. | q.s. | q.s. | q.s. | q.s. | q.s. | q.s. | q.s. | q.s. | q.s. |
| Purified water | Balance | Balance | Balance | Balance | Balance | Balance | Balance | Balance | Balance | Balance | Balance |

| | | | | | | | | | | | |
|---|---|---|---|---|---|---|---|---|---|---|---|
| *1 Lecinol S-10E (iodine value: not more than 10); product of Nikko Chemicals Co., Ltd. *2 SLP-PC92H (iodine value: not more than 45); product of Tsuji Oil Mill Co., Ltd. *3 BASIS LS-60H (iodine value: not more than 10); product of The Nisshin Oi11i0 Group, Ltd. | | | | | | | | | | | |

### INDUSTRIAL APPLICABILITY

The composition for external use of the present invention is capable of improving the percutaneous absorption of bioactive components, due to the phospholipid (i) and at least 22 wt.% of mono- or oligo-glycol ether (ii) contained therein.

Further, the composition of the present invention enables efficient percutaneous absorption of bioactive components due to 0.01 to 8 wt.% of phospholipid (i) and 0.01 to 6.5 wt.% of mono- or oligo-glycol ether (ii) contained therein.

Furthermore, the composition of the present invention is capable of improving the percutaneous absorbability of (iv) at least one bioactive component selected from the group consisting of hyaluronic acid, hyaluronic acid derivatives, vitamin A, vitamin A derivatives, vitamin C, specific vitamin C derivatives, xanthine derivatives, ubiquinones, and salts thereof, due to the phospholipid (i) and mono- or oligo-glycol ether (ii) contained therein. The composition of the present invention is particularly advantageous in that it can impart excellent percutaneous absorbability to hyaluronic acid, which is not readily absorbed into the skin.

## Claims

1. A composition for external use comprising (i) a phospholipid and (ii) a mono- or oligo-glycol ether, wherein the proportion of mono- or oligo-glycol ether (ii) is at least 22 wt.% of the total amount of the composition.

2. A composition according to claim 1, further comprising (iii) a bioactive component.

3. A composition according to claim 1, wherein the mono- or oligo-glycol ether (ii) is a compound represented by Formula (1):
R₁-O- (A-O) ₙ R₂ (1)
wherein R₁ and R₂ are the same or different, and each independently represent a C₁₋₆ alkyl group, a C₂₋₆ alkenyl group, an aryl group, an acetyl group, or a hydrogen atom, provided that at least one of R₁ and R₂ represents a C₁₋₆ alkyl group, a C₂₋₆ alkenyl group, or an aryl group;
A represents a C₂₋₄ alkylene group; and
n represents an integer of 1 to 4.

4. A composition according to claim 1, wherein the proportion of phospholipid (i) is 0.01 to 15 wt.% of the total amount of the composition.

5. A composition for external use comprising (i) a phospholipid in a proportion of 0.01 to 8 wt.%, and (ii) a mono-or oligo-glycol ether in a proportion of 0.01 to 6.5 wt.%, based on the total amount of the composition.

6. A composition according to claim 5, the further comprising (iii) a bioactive component.

7. A composition according to claim 5, wherein the mono- or oligo-glycol ether (ii) is a compound represented by Formula (1):
R₁-O-(A-O)ₙ-R₂ (1)
wherein R₁ and R₂ are the same or different, and each independently represent a C₁₋₆ alkyl group, a C₂₋₆ alkenyl group, an aryl group, an acetyl group, or a hydrogen atom, provided that at least one of R₁ and R₂ represents a C₁₋₆ alkyl group, a C₂₋₆ alkenyl group, or an aryl group;
A represents a C₂₋₄ alkylene group; and
n represents an integer of 1 to 4.

8. A composition for external use comprising (i) a phospholipid, (ii) a mono- or oligo-glycol ether, and (iv) at least one bioactive component selected from the group consisting of hyaluronic acid, vitamin A, vitamin A derivatives, ascorbic acid, salts of ascorbic acid, dehydroascorbic acid, ascorbyl glucoside, ascorbyl stearate, L-ascorbyl dipalmitate, ascorbyl tetra-2-hexyldecanate, xanthine derivatives, ubiquinones, and salts thereof.

9. A composition according to claim 8, wherein the mono- or oligo-glycol ether (ii) is a compound represented by Formula (1):
R₁-O-(A-O)ₙ-R₂ (1)
wherein R₁ and R₂ are the same or different, and each independently represent a C₁₋₆ alkyl group, a C₂₋₆ alkenyl group, an aryl group, an acetyl group, or a hydrogen atom, provided that at least one of R₁ and R₂ represents a C₁₋₆ alkyl group, a C₂₋₆ alkenyl group, or an aryl group;
A represents a C₂₋₄ alkylene group; and
n represents an integer of 1 to 4.

10. A composition according to claim 8, wherein the bioactive component (iv) is hyaluronic acid and/or a salt thereof.

11. A composition according to claim 8, wherein the bioactive component (iv) is at least one member selected from the group consisting of retinol, retinol acetate, retinol palmitate, retinal, retinoic acid, methyl retinoate, ethyl retinoate, retinol retinoate, vitamin A fatty acid esters, d-δ-tocopheryl retinoate, α-tocopheryl retinoate, and β-tocopheryl retinoate.

12. A composition according to claim 8, wherein the bioactive component (iv) is at least one member selected from the group consisting of ascorbic acid, salts of ascorbic acid, dehydroascorbic acid, ascorbyl glucoside, ascorbyl stearate, L-ascorbyl dipalmitate, and ascorbyl tetra-2-hexyldecanoate.

13. A composition according to claim 8, wherein the bioactive component (iv) is at least one xanthine derivative selected from the group consisting of caffeine, aminophylline, theophylline, oxtriphylline, dyphylline, diisobutylaminobenzoyloxypropyl theophylline, theobromine, diprophylline, proxyphylline, and pentoxifylline.

14. A composition according to claim 8, wherein the bioactive component (iv) is at least one ubiquinone selected from the group consisting of coenzymes Q6, Q7, Q8, Q9, and Q10.

15. Use of a composition for external use comprising (i) a phospholipid, and (ii) a mono- or oligo-glycol ether in a proportion of at least 22 wt.% of the total amount of the composition, for the manufacture of a preparation in which percutaneous absorption of (iii) a bioactive component is improved.

16. Use of a composition for external use comprising (i) a phospholipid in a proportion of 0.01 to 8 wt.%, and (ii) a mono- or oligo-glycol ether in a proportion of 0.01 to 6.5 wt.%, based on the total amount of the composition, for the manufacture of a preparation in which percutaneous absorption of (iii) a bioactive component is improved.

17. Use of a composition for external use comprising (i) a phospholipid and (ii) a mono- or oligo-glycol ether, for the manufacture of a preparation in which percutaneous absorption of (iv) at least one bioactive component selected from the group consisting of hyaluronic acid, vitamin A, vitamin A derivatives, ascorbic acid, salts of ascorbic acid, dehydroascorbic acid, ascorbyl glucoside, ascorbyl stearate, L-ascorbyl dipalmitate, ascorbyl tetra-2-hexyldecanoate, xanthine derivatives, ubiquinones, and salts thereof, is improved.
